# EUROPÄISCHE PATENTSCHRIFT

(11) **EP 3 439 466 B1**
(45) Veröffentlichungstag und Bekanntmachung des Hinweises auf die Patenterteilung: **07.09.2022**
(21) Anmeldenummer: 17717087.5
(22) Anmeldetag: 20.03.2017
(51) Int. Cl.: A01K 45/00, G01N 21/64, G01N 33/08, A01K 41/00, A01K 43/00

(54) **VERFAHREN UND VORRICHTUNG ZUR OPTISCHEN IN-OVO GESCHLECHTSBESTIMMUNG VON BEFRUCHTETEN UND BEBRÜTETEN VOGELEIERN**
METHOD AND DEVICE FOR OPTICAL IN OVO SEX DETERMINATION OF FERTILIZED AND INCUBATED BIRDS' EGGS
PROCÉDÉ ET DISPOSITIF DE SEXAGE VISUEL IN-OVO D'OEUFS D'OISEAUX FÉCONDÉS ET INCUBÉS

(30) Priorität: 04.04.2016 DE 102016004051
(43) Veröffentlichungstag der Anmeldung: 13.02.2019
(73) Patentinhaber: Technische Universität Dresden, 01069 Dresden (DE)
(72) Erfinder: STEINER, Gerald, 08340 Schwarzenberg (DE); PREUSSE, Grit, 01445 Radebeul (DE); GALLI, Roberta, 01309 Dresden (DE); KOCH, Edmund, 01307 Dresden (DE)
(74) Vertreter: Rauschenbach, Marion
(86) Internationale Anmeldenummer: PCT/EP2017/056536
(87) Internationale Veröffentlichungsnummer: WO 2017/174337

(56) Entgegenhaltungen:
- EP-A1- 2 336 751
- DE-A1-102014 010 150
- M. AAMIR ASLAM ET AL: "A reliable method for sexing unincubated bird eggs for studying primary sex ratio", MOLECULAR ECOLOGY RESOURCES, Bd. 12, Nr. 3, 1. Mai 2012 (2012-05-01), Seiten 421-427, XP055035399, ISSN: 1755-098X, DOI: 10.1111/j.1755-0998.2012.03120.x
- Brigitte Osterath: "Forscher blicken ins Hühnerei", , 17. April 2014 (2014-04-17), XP055376659, Gefunden im Internet: URL:http://www.dw.com/de/forscher-blicken- ins-hühnerei/a-17015417 [gefunden am 2017-05-29]
- Susanne Dammers ET AL: "Raman-Spektroskopie", , 6. Juni 2005 (2005-06-06), XP055376676, Gefunden im Internet: URL:http://www.uni-muenster.de/imperia/md/ content/physikalische_chemie/praktikum/ram an_dammers__doedt.pdf [gefunden am 2017-05-29]
- Kerstin Reiners ET AL: "Infrarot-und Ramanspektroskopie", , 1. Januar 2003 (2003-01-01), XP055376677, Gefunden im Internet: URL:http://www.greco.uni-oldenburg.de/Pape r/InfrarotRamanVortrag.pdf [gefunden am 2017-05-29]

## Beschreibung

Die Erfindung betrifft ein Verfahren und eine Vorrichtung zur optischen in-ovo Geschlechtsbestimmung von befruchteten und bebrüteten Vogeleiern.

In der Druckschrift DE 10 2014 010 150 A1 und WO 2016000678 A1 ist ein Verfahren zur Ramanspektroskopischen in-ovo Geschlechtsbestimmung von befruchteten und bebrüteten Vogeleiern beschrieben, wobei der Embryo einschließlich der extraembryonalen Strukturen beweglich im Ei ist, und zum Zeitpunkt einer Messung noch nicht an der Kalkschale fixiert ist. Dabei werden folgende Schritte durchgeführt:
- Überwachung des Zeitverlaufs des Bebrütens bis zur Ausbildung mindestens eines erkennbaren Blutgefäßes,
- Schaffung eines Lochs in der Kalkschale im Nahbereich des erkannten Blutgefäßes mittels einer Locherzeugungseinheit;
- Suchen der sich im Ei ausbildenden Blutgefäße mittels eines Visions-Systems und einer koaxialen oder lateralen Beleuchtung mit Licht des sichtbaren Wellenlängenbereiches,
- Positionieren zumindest eines Blutgefäßes in den Laserfokus einer Laserquelle entweder durch Bewegen des Eis oder Bewegen eines Objektivs einer Vorrichtung zur Einbringung des Laserlichts und Erfassung der Ramanstreustrahlung,
- Registrieren der Ramanstreustrahlung des bestrahlten Blutgefäßes mittels der Vorrichtung zur Einbringung des Laserlichts und zur Erfassung der Ramanstreustrahlung, wobei während der Messung eine Bewegung des Blutgefäßes aus dem Fokus heraus durch Nachführung mittels des Visions-Systems vermieden werden kann,
- Auswertung der Ramanstreustrahlung und Bestimmung des Geschlechts in einer Auswerteeinheit,
- Anzeige des Geschlechtes des Embryos im Vogelei.

Die Ramanspektren werden in der Form korrigiert, dass Untergrundsignale durch Fluoreszenz oder andere Streuprozesse eliminiert werden und die Spektren in vorgegebener Form normiert werden, bevor die mathematische Analyse mittels Verfahren der gestützten und nicht gestützten Klassifizierung erfolgt.

In den Druckschriften US 8 364 247 B2 und EP 2 336 751 A1 wird ein Verfahren zur Bestimmung des Geschlechts von Vogeleiern beschrieben, bei dem mit einer Strahlungsquelle elektromagnetische Strahlung auf die Keimscheibe eines Eies emittiert und nach dem Abschalten der Strahlungsquelle am bestrahlten Bereich der Keimscheibe das Abklingverhalten der angeregten Eigenfluoreszenzintensität zeitaufgelöst und spektralaufgelöst für mindestens eine Wellenlänge der Eigenfluoreszenz mit einem Detektor erfasst wird. Mit den ermittelten Intensitätsmesswerten wird die fraktale Dimension berechnet und der Wert der fraktalen Dimension DF mit einem art- und geschlechtsspezifischen Grenzwert verglichen; wobei bei Überschreiten des Grenzwertes das jeweilige Ei als weiblich und bei Unterschreiten des Grenzwertes als männlich eingestuft wird.

Das Verfahren wird an Keimscheiben befruchteter Vogeleier, also am Tag "Null" der Bebrütung, angewendet. Der Nachteil des Verfahrens besteht darin, dass die Ei-Öffnung am Tag "Null" zu stark reduzierten Schlupfraten führt. Es besteht weiterhin die Gefahr einer möglichen irreparablen Schädigung der Keimscheibe durch die eingesetzte UV-Strahlung bei 337 nm.

In der Druckschrift US 7 950 349 B1 wird ein Verfahren zur Bestimmung
1) der Fruchtbarkeit eines Vogeleies durch Messung der Lumineszenz und der Biophotonenintensität (Photonen pro Sekunde) des Eies nach Einwirkung einer externen Lichtquelle und
2) des Geschlechtes eines Vogeleies durch Messen des Photonenspektrums der Biophotonenemission und Lumineszenz des Eies nach Einwirkung einer externen Lichtquelle
   beschrieben.

Dabei kann die externe Lichtquelle entweder eine Glühlampe, Leuchtstofflampe, LED oder eine (gepulste oder CW) monochromatische Laserlichtquelle oder dichromatische Laserlichtquelle sein. Der erste Teil des Verfahrens ergibt sich aus der Tatsache, dass nach Exposition mit der Lichtquelle, die fruchtbaren Vogeleier eine höhere Intensität der Photonen als die der unbefruchteten Vogeleier emittieren. Der zweite Teil des Verfahrens ergibt sich aus der Tatsache, dass nach Exposition mit den genannten Lichtquellen, Vogeleier des weiblichen Geschlechts ein anderes Spektrum von Photonen als Vogeleier des männlichen Geschlechts emittieren.

Die Nachteile des Verfahrens sind:
- Die Lumineszenz ist extrem schwach und die gemessenen Intensitäten betragen nur wenige bis einige hundert Photonen pro Sekunde und pro Quadratzentimeter Oberfläche zwischen 200 und 800 nm, wie in der Druckschrift Popp, Fritz: "Properties of biophotons and their theoretical implications", Indian Journal of Experimental Biology 41, 2003, S. 391-402 beschrieben ist.
- Dabei ist eine lange Expositionszeit notwendig (30 s mit Halogenlampe), wie in der Druckschrift Michael A. Grashorn, Ulrike Egerer: INTEGRATED AS-SESSMENT OF QUALITY OF CHICKEN ORGANIC EGGS BY MEASURE-MENT OF DARK LUMINESCENCE Pol. J. Food Nutr. Sci. 2007, Vol. 57, No. 4(A), pp. 191-194 beschrieben ist.

In den Druckschriften WO 2010/150265 A3 und US 2013/0044210 A1 wird ein Verfahren zur Geschlechtsbestimmung unbebrüteter Vogeleier durch hyperspektrale Analyse optischer Spektren, insbesondere Reflexionsspektren beschrieben. Die Analyse erfolgt in einem Spektralbereich mit Wellenlängen bis zu 2500 nm (MIR), um das vom Kalziumkarbonat CaC0₃ der Kalkschale des Eies erzeugte Signal bei 2340 nm herausfiltern zu können. Das Verfahren ermöglicht es, biologische Komponenten, andere als Blut, zu detektieren und ermöglicht sowohl die Erfassung der Fruchtbarkeit vor dem zweiten Bebrütungstag als auch die Bestimmung des Geschlechts der Küken im Ei am zwölften Bebrütungstag. Die Sensitivität kann durch die Verwendung einer neuronalen Netzwerkanalyse erhöht werden. Mittels der Hauptkomponentenanalyse (PCA) werden die spektralen Merkmale bestimmt, die für die Varianzen zwischen den unbefruchteten Kontrolleiern und den Probeneiern verantwortlich sind. Mittels einer neuronalen Netzwerkanalyse auf der Grundlage der PCA-Ergebnisse werden dann die kleinen, aber signifikanten Veränderungen zwischen den Kontrolleiern und den experimentellen Eiern erhalten. Das Verfahren ermöglicht die Bestimmung der Fruchtbarkeit mit mehr als 90% Genauigkeit am Tag "Null" (Tag der Eiablage) und des Geschlechtes der Küken mit mehr als 75% Genauigkeit am zwölften Bruttag.

Ein Nachteil besteht darin, dass mit dem Verfahren eine Geschlechtsbestimmung erst am 12. Tag durchgeführt werden kann.

In der Druckschrift DE 10 2007 013 107 B4 wird ein DNA relevantes Zellmaterial durch Schwingungsspektroskopie analysiert, um das Geschlecht von Vogeleiern anhand von DNA-Unterschieden zu bestimmen: Entweder im Ei nach Öffnung mit einer Sonde oder nach Entnahme des Materials aus dem Ei und Deposition auf einem Substrat. Die UV-Resonanzraman-Spektroskopie wird bei Wellenlängen von 244 nm oder 254 nm festgelegt. Dabei wird eine Spektrenklassifikation mit allen existierenden überwachten und nichtüberwachten Verfahren durchgeführt:
- mit einem DNA-relevanten Material mit speziellem Bezug zur Federpulpa und der Keimscheibe und
- mit einer UV-Raman-Spektroskopie bei einer Wellenlänge von 244 nm oder 257 nm.

Bei dem Verfahren wird somit DNA-relevantes Zellmaterial des geschlechtlich zu bestimmenden Vogels mit Licht untersucht und die Molekülschwingungen gemessen, wobei das durch das Licht entstehende Spektrum der Molekülschwingungen erfasst und mit vorgegebenen sowie geschlechtsspezifische DNA-Strukturen der zu untersuchenden Vogelart repräsentierenden Referenzspektren verglichen wird und wobei aus diesem Spektralvergleich eine auf Grundlage des DNA-Gehalts des Zellmaterials basierende Geschlechtszuordnung des Vogels getroffen wird.

Die Molekülschwingungen werden dabei mittels Anwendung der Raman-Spektroskopie oder der IR-Spektroskopie gemessen, wobei z.B. das DNArelevante Zellmaterial aus dem Schaft einer jungen Feder eines Vogels entnommen werden kann. Das Zellmaterial wird auf einem Träger präpariert und mit Licht abgetastet. In einem anderen in der Druckschrift DE 10 2007 013 107 A1 beschriebenen Teilverfahren wird das Licht zur Messung der Molekülschwingungen des DNA-relevanten Zellmaterials von ungeschlüpften Vögeln durch die Eierschale hindurch auf den Embryo oder die Keimscheibe fokussiert, wobei das Spektrum der durch die Molekülschwingungen entstehenden Strahlung im Ei mit einer durch dessen Schale hindurch geführten Sonde gemessen wird.

Für die Hindurchführung der Sonde wird zur Messung des Spektrums wenigstens ein mikroskopisch kleines Loch durch die Eischale hindurch gebohrt. Das Licht wird durch den kleinen Zugang durch die Eischale unmittelbar auf die Keimscheibe als Zellmaterial fokussiert. Durch den gleichen oder einen anderen Zugang geringer Öffnungsgröße wird die Sonde eingeführt, mittels der das reflektierte und von der Sonde aufgenommene Spektrum der vorgenannten Molekülbewegung im Innern des Eis gemessen wird.

Die erhaltenen spektralen Informationen werden in einem zweiten Schritt mit geschlechtsspezifischen Referenzdaten verglichen und einem Klassifizierungsalgorithmus zugeführt. Diese repräsentieren vorzugsweise statistisch gewonnene Daten über die zu untersuchenden Vogelspezies. Aus diesem Vergleich wird die Geschlechtszuordnung des zu untersuchenden DNA-Materials getroffen.

Ein Problem besteht darin, dass für die Einbringung einer Sonde in vorgefertigte Löcher bei einer sehr großen Anzahl von zu untersuchenden Vogeleiern ein großer zeitlicher Aufwand erforderlich ist. Außerdem muss bei einer Fokussierung des Lichts aus der Sonde auf die Keimscheibe ein erheblicher Justierungsaufwand für eine optische Abbildung in Bezug auf den Ort der Keimscheibe betrieben werden, wobei von Ei zu Ei die Fokussierungsebene eine andere Lage aufweisen kann und damit keine Bestimmung des Geschlechts durchgeführt werden kann.

Problematisch an diesen Untersuchungen ist weiterhin, dass das zur Untersuchung der Keimscheiben notwendige Einbringen der Löcher in die Kalkschale einschließlich der Eischalenmembran am Tag "Null" zu einer Beeinträchtigung der Emryonalentwicklung und zu stark sinkenden Schlupfraten führt, wie in den Druckschriften S. Klein: Analysis of chicken embryonic development after removal of blastodermal cells for sexing. British Poultry Science (39), 1998, S. 482-487; einschließlich darin zitierter Literatur: J. Brake, T. W. (1997). Egg handling and storage. Poultry science (76), S. 144-151 beschrieben ist.

Als Datenvorbehandlung wird eine Ableitung der 1. Ordnung sowie eine Vektornormierung durchgeführt, wobei alle Informationen über die Fluoreszenz eliminiert werden.

In der Druckschrift DE 10 2010 006 161 B3 sind ein Verfahren und eine Vorrichtung zur Bestimmung des Geschlechts von befruchteten und nicht bebrüteten Vogeleiern beschrieben, wobei ein Ei zumindest eine feste Kalkschale, ein von der Kalkschale und weiteren Eihüllen umgebenes Eidotter und eine dem Eidotter zugeordnete Keimscheibe enthält, wobei eine Sonde zur Messung eines Spektrums durch ein Loch der Kalkschale hindurch in Richtung zur Keimscheibe mit Keimscheibenzellen geführt wird, wobei das Verfahren folgende Schritte aufweist:
- Positionierung der Sonde im Bereich der Keimscheibe,
- spektroskopische in-ovo Charakterisierung der Keimscheibenzellen,
- Erkennung des Geschlechts durch eine automatische Klassifizierung der reflektiven Spektren,
wobei als Sonde ein optischer Kristall eingesetzt wird, mit dem eine schnelle und rückwirkungsfreie Aufnahme eines Infrarot- und/oder Nahinfrarotspektrums bei Nutzung der abgeschwächten Totalreflexion innerhalb des optischen Kristalls durch das evaneszente Feld im Bereich der Keimscheibe durchgeführt wird, wobei die Extinktion infolge einer spektralen Absorption von geschlechtsspezifischen Keimscheibenzellen erfolgt,
wobei die Positionierung des optischen Kristalls durch eine permanente automatische Auswertung der rückgeführten Spektren bis zur Bestimmung von geschlechtsspezifischen Keimscheibenzellen begleitet wird, bis das Spektrum ausgewertet und das Geschlecht des befruchteten Eies eindeutig angezeigt wird.

Während des Positionierungsvorgangs werden permanent rückgeführte IR- und/oder NIR-Spektren aufgezeichnet und einer Auswertung zugeführt, wobei eine automatische Klassifizierung der Spektren anhand des spektralen Fingerabdruckes zum Beispiel in Proteinen, Lipiden und Nukleinsäuren erfolgt.

Bei der geschlechtsspezifischen Absorption des einfallenden IR- und/oder NIR-Lichts werden die Keimscheibenzellen anhand von Absorptionsbanden der Nukleinsäuren (DNA und RNA) sowie weiterer biochemischer Verbindungen derart identifiziert, dass das Geschlecht des geprüften Eies bestimmt und angezeigt wird.

Die Messung kann mit einer herkömmlichen Infrarot-Spektroskopie durchgeführt werden.

Die in der Druckschrift DE 10 2010 006 161 B3 beschriebene zugehörige Vorrichtung enthält
- mindestens eine Eipositions-Auflage zur Arretierung mindestens eines Eies,
- mindestens eine Höhenverstelleinrichtung mit mindestens einem Haltearm,
- mindestens einen als Sonde ausgebildeten optischen Kristall, der an dem Haltearm befestigt ist,
- mindestens eine Steuereinheit für die eiarretierende Eipositions-Auflage und für die Höhenverstelleinrichtung,
- mindestens eine auf mindestens einen Wellenlängenbereich bezogene spektrale Lichtquelle, die einen IR- und/oder NIR-Lichtstrahl aussendet,
- mindestens einen Detektor zur Aufnahme des rückgeführten IR- und/oder NIR-Lichtstrahls,
- mindestens ein optisches Element zur geführten Strahlführung zwischen der Lichtquelle und dem optischen Kristall und zur rückgeführten Strahlführung vom optischen Kristall hin zum Detektor, sowie
- eine mit dem Detektor verbundene Auswerteeinheit und eine Anzeigeeinheit, wobei mit der Höhenverstelleinrichtung die Höhe des Haltearms und somit des optischen Kristalls in Bezug auf den Ort der Keimscheibe einstellbar ist und der optischen Kristall im Bereich der Keimscheibe in einer Scheibenzuordnungsposition positionierbar ist, in der über den optischen Kristall ein sich bei Totalreflexion an der zur Keimscheibe gerichteten Ausgangsfläche ausbildendes evaneszentes Feld auf die Keimscheibe übergreift und die darin befindlichen Keimscheibenzellen wechselwirkend mit dem evaneszenten Feld eine geschlechtsspezifische Absorption des Licht aus dem einfallenden Strahlengang vornehmen, wobei das an der Ausgangsfläche totalreflektierte Licht über den rückgeführten Strahlengang innerhalb des Kristalls und schließlich über das optische Element zur Registrierung zum Detektor geführt ist, von dem aus die registrierten spektralen Signale zur Auswertung und Anzeige des Geschlechts übermittelt werden.

In der Druckschrift WO 2014/021715 A2 wird eine Geschlechtsbestimmung von Vogelembryonen beschrieben, wobei das Verfahren durchgeführt wird mittels
a) Detektion einer Markerverbindung von Zuckern und Aminosäuren, Vorstufen und Metaboliten in der Allantoisflüssigkeit des Eies am 8.-11. Bruttag,
b) Quantitativer Bestimmung des Markers mittels NMR-Spektroskopie,
c) Geschlechtsbestimmung durch Vergleich der Menge des Markers zu einem vorgegebenen Ausgangswert.

Dabei erfolgen
- eine Bestimmung der absoluten Mengen oder von Mengenverhältnissen von Verbindungen (Glukose, Cholin, Valin), um sie mit einem Basiswert zu vergleichen, und
- eine Anwendung von unsupervised chemometrischen Verfahren (wie leastsquare modeling oder PCA) auf quantitative Mengen oder Mengenverhältnissen zur Geschlechtsbestimmung.

Ein wesentlicher Nachteil des Verfahrens besteht darin, dass eine Entnahme mindestens einer Probe aus dem Ei erfolgt.

In den Druckschriften WO 2010/1031 11 A1 und US 2012/0058052 A1 werden ein nicht-invasives Verfahren und eine Vorrichtung zur in-ovo-Bestimmung des Geschlechts von Vogelarten beschrieben. Das Verfahren umfasst die Schritte der Einführung eines markierten Antikörpers in das Ei, der sich an ein geschlechtsspezifisches Antigen des Embryos anbindet, und die Detektion des markierten gebundenen Antikörpers mit einer Erfassungseinrichtung außerhalb des Eies.

In der Druckschrift US 7 041 439 B2 werden ein Verfahren und eine Vorrichtung für die automatisierte Prozessführung von Eiern nach ausgewählten Charakteristika (z.B. des Geschlechtes) beschrieben, wobei folgende Schritte ablaufen:
a) Extraktion von Probenmaterial (Allantoisflüssigkeit, Eiweiß, Eigelb, Eischale, Albumin, Gewebe, Membran und / oder Blut),
b) Analyse des extrahierten Materials zur Bestimmung der ausgewählten Charakteristika und
c) selektive Prozessführung der identifizierten Eier.

Beispielsweise wird darin ein Verfahren zur Verarbeitung von Eiern auf der Basis des Geschlechts dargestellt, welches folgende Schritte aufweist:
1) Identifizierung lebender Eier,
2) Extraktion von Allantoisflüssigkeit aus den als lebend identifizierten Eiern,
3) Ermittlung des Östrogengehaltes und einer Farbveränderung in der extrahierten Allantois-Flüssigkeit zur Geschlechtserkennung,
4) selektive Injektion eines Impfstoffs in Abhängigkeit des Geschlechtes.

Ein Nachteil besteht darin, dass die Untersuchung der Allantoisflüssigkeit am Tag 13 bis 18 erfolgt. Auch hier ist eine Probenentnahme notwendig.

In der Druckschrift US 6 365 339 B1 ist ein Verfahren zur Geschlechtsbestimmung von Vogelembryonen beschrieben, bei dem während des Brutprozesses nach Aufbohren der Kalkschale Proben von der Allantoisflüssigkeit des Embryos genommen und in einem lonenmobilitätsspektrometer (IMS) analysiert werden. Die resultierenden Spektren enthalten relevante Markerpeaks, die mit geschlechtsspezifischen Mobilitäten korrelieren.

Ein Nachteil besteht darin, dass auch hier Proben entnommen werden müssen, die einen höheren Aufwand zumindest im Umfeld zur Geschlechtsbestimmung bedingen.

In der Druckschrift US 6 029 080 B1 werden ein nichtinvasives Verfahren und eine Vorrichtung zur Geschlechtsbestimmung von Vogeleiern dargestellt, bei dem mittels magnetischer Kernresonanz (NMR) bestimmt wird, ob der lebende Embryo im Ei männliche Geschlechtsorgane oder weibliche Geschlechtsorgane aufweist.

Ein Nachteil besteht darin, dass die Ausbildung der Geschlechtsorgane erst im entwickelten Embryo nach wesentlichen mehreren Tagen erfolgt, wobei zumindest die Durchführung eines hohen finanziellen Aufwandes bedarf.

In der Druckschrift US 6 506 570 B1 wird zur in-ovo Geschlechtsbestimmung von Vogeleiern die Anwesenheit oder Abwesenheit eines erhöhten geschlechtsspezifischen Hormonspiegels, vorzugsweise des Östrogenspiegels, in einer extraembryonalen Flüssigkeit, vorzugsweise der Allantoisflüssigkeit, bestimmt. Das Verfahren wird vorzugsweise auf Hühnereier angewendet und kann vor oder während der Umlagerung aus dem Vorbrüter in den Schlupfschrank durchgeführt werden.

In der Druckschrift DE 10 2012 023 947 A1 ist ein Verfahren zur Strukturaufklärung durch eine optisch undurchsichtige Barriere eines biologischen Untersuchungsobjektes hindurch beschrieben. Bei dem Untersuchungsobjekt wird eine innere Struktur mit unterschiedlichen dielektrischen Eigenschaften mittels elektromagnetischer Spektralanalyse aufgeklärt, wobei das Untersuchungsobjekt unter einem Array von Pulssendern und Empfängern, welche in einer Ebene angeordnet und daten- sowie informationsleitend mit einem Computersystem verbunden sind, positioniert wird,
wobei durch die Pulssender elektromagnetischen Pulse im Spektralbereich 0,01 THz bis 1 THz auf das positionierte Untersuchungsobjekt ausgesendet werden, wobei die vom Untersuchungsobjekt ausgehende Strahlung von den Empfängern aufgenommen und dem Computer über datenleitende sowie informationsleitende Verbindungen für ein bildgebendes Verfahren zugeleitet wird.

Da die THz-Strahlung sehr schwach ist, treten lange Messzeiten auf, die zur Durchführung einer schnellen, automatischen Geschlechtsbestimmung hinderlich sind. Außerdem erfordert eine starke Absorption von begleitendem Wasserdampf im THz-Bereich eine extrem niedrige bzw. konstante Luftfeuchtigkeit in der Brüterei, was wiederum einen hohen technischen Zusatzaufwand bedeutet.

In der Druckschrift DE 20 2013 011 765 U1 ist eine spektralphotometrische Analyse der Federfarbe von Hühnerembryos beschrieben. Dabei wird elektromagnetische Energie mit einer Wellenlänge zwischen etwa 380 nm und 740 nm zur nicht invasiven Bestimmung der Geschlechts von Vogelembryos verwendet, wobei ein Vogelei der elektromagnetischen Energie ausgesetzt und die Menge an Absorption, Diffusion, Refraktion, Reflexion oder einer beliebeigen Kombination davon der elektromagnetischen Energie durch das Vogelei bestimmt wird. Es wird über das Vorliegen oder Fehlen von Farbpigment im Inneren des Vogeleies das Geschlecht des Vogelembryos wenigstens teilweise bestimmt.

Die spektralphotometrische Analyse der Federfarbe von Hühnerembryos kann nur auf braue Rassen bzw. auf Rassen mit Farbunterschied von weiblichen Küken oder männlichen Küken angewendet werden.

Im Folgenden wird eine Zusammenfassung der Nachteile der Verfahren aus den genannten Druckschriften angegeben:
1. die Geschlechtsbestimmung am Tage "Null" bedingt einen Zugang zur festgestellten Position der Keimscheibe, was nach bisherigen Erfahrungen eine Beeinträchtigung der Embryonalentwicklung und stark sinkende Schlupfraten mit sich bringen,
2. bei der späten Geschlechtbestimmung mit Inkubationstagen von 7 bis 21 Tagen spielen
   erstens tierschutzrechtliche Aspekte,
   - wobei ab dem 7. Inkubationstag das Schmerzempfinden des Vogelembryos beginnt und
   - wobei ein spätes Abtöten weit entwickelter Vogelembryonen durchgeführt wird, weil mit steigender Inkubation der Ei-Inhalt aus dem Vogelembryo selbst besteht, und
   zweitens ökonomische Aspekte
   - wobei bei der späten Geschlechtsbestimmung die männlichen Eier länger im Brutschrank sind, was eine weitgehend schlechtere Auslastung der Brutschränke und somit höhere Stromkosten bedingen
   eine wesentliche Rolle.
3. Bei einer Geschlechtsbestimmung mit einer Entnahme von Probenmaterial können folgende Probleme auftreten:
   - die nach jeder Messung notwendige zusätzliche Reinigung und Desinfektion bzw. Ersatz von Geräten oder Geräteteilen (z.B. Kanülen) erhöht laufende Verbrauchskosten deutlich,
   - es ergibt sich eine erschwerte Automatisierbarkeit gegenüber kontaktlosen Verfahren und
   - das Infektionsrisiko wird stark erhöht, so dass sich eine Gefahr der reduzierten Schlupfraten ergeben kann.

Im Folgenden wird beschrieben, wie bisher eine endogene Fluoreszenz des Blutes - unabhängig von der Geschlechtsbestimmung - genutzt wird, um Blut zu charakterisieren:
In der Druckschrift US 2011/053210 ist ein Blutanalysator beschrieben, der auf Fluoreszenzmessung basiert.

Der Blutanalysator umfasst
- einen Probenvorbereitungsabschnitt,
- eine Messprobe aus einer Blutprobe und aus einem hämolytischen Mittel,
- eine Lichtinformationseinheit, die die Fluoreszenzinformationen und zumindest zwei Typen von Streulichtinformationen von der Messprobe erzeugt und
- eine Steuereinheit für eine erste Klassifizierung der weißen Blutkörperchen in der Messprobe in mindestens vier Gruppen (u.a. Monozyten, Neutrophile, Eosinophile) auf der Grundlage der Informationen aus den Fluoreszenzsignalen und den Signalen der zwei Arten des gestreuten Lichtes.

Nachteile bestehen darin,
dass die Blutanalyse keine Informationen über das Geschlecht, sondern nur Informationen über die Blutzellen ergibt. Die Blutanalyse erfolgt nicht in situ im Blutgefäß, sondern auf separierten Blutproben. Die Anregungswellenlänge liegt zwischen 350 nm und 500 nm. Die Signale werden nicht spektroskopisch ausgewertet, es erfolgt nur eine Auswertung der Intensität des rückgestreuten und transmittierten Lichtes.

In der Druckschrift US 2014/332697 ist ein Verfahren für spektrale Detektionsverfahren von Blutkomponenten nach Thalassämie beschrieben, welches auf Fluoreszenzspektren von Biomolekülen basiert. Zu den Biomolekülen gehören u.a. Tyrosin, Tryptophan, Nicotinamid-Adenin-Dinucleotid und Flavin-Adenin-Dinukleotid, die alle im Blutplasma gefunden werden sowie Porphyrin aus den Erythrozyten. Bei diesem Verfahren werden die Verhältnisse der Intensitätsmaxima zwischen Tryptophan, Nicotinamid-Adenin-Dinucleotid, Flavin-Adenin-Dinucleotid, Nicotinamid-Adenin-Dinucleotid, Tyrosin Tryptophan und Porphyrin verwendet, um einen Patienten mit Thalassämie zu diagnostizieren.

Die Nachteile bestehen darin, dass
die Analyse nicht in situ im Blutgefäß, sondern auf separierten Blutproben erfolgt. Die Anregung erfolgt im UV-Bereich, die Registrierung der Fluoreszenzsignale im UV und VIS-Bereich zwischen 350 nm und 500 nm.

In der Druckschrift US 2014/0308697 A1 sind Verfahren und Vorrichtungen zur Identifizierung von durch Plasmodium infizierten Erythrozyten beschrieben. Die Verfahren umfassen:
- Erhalten eines vorwärtsgestreuten Lichtsignales, eines Seitenstreulichtsignales und eines optionalen Fluoreszenzsignales von Zellen in einer Blutprobe;
- Erhalten eines zweidimensionalen Scattergrammes (Vorwärtsstreulichtsignal und Seitenstreulichtsignal) oder ein dreidimensionales Scattergrammes (Vorwärtsstreulichtsignal, Seitenstreulichtsignal und Fluoreszenzsignal);
- Identifizierung von durch Plasmodium infizierte Erythrozyten als Zellen, die sich in einem vorbestimmten Bereich des zweidimensionalen Scattergrammes oder des dreidimensionalen Scattergrammes befinden.

Der Erfindung liegt die Aufgabe zugrunde, ein Verfahren und eine Vorrichtung zur optischen in-ovo Geschlechtsbestimmung von befruchteten und bebrüteten Vogeleiern anzugeben, die derart geeignet ausgebildet sind, dass das Geschlecht bereits in den Eiern schnell und zuverlässig eindeutig bestimmt werden kann, Es soll sich der weibliche Embryo normal weiterentwickeln und es soll zum Schlupf des weiblichen Kükens kommen.

Die Aufgabe wird durch die Merkmale der Patentansprüche 1 und 14 gelöst.

In dem Verfahren zur optischen in-ovo Geschlechtsbestimmung von befruchteten und bebrüteten Vogeleiern werden folgende Schritte durchgeführt:
- Überwachung des Zeitverlaufs des Bebrütens bis zur Ausbildung mindestens eines erkennbaren Blutgefäßes mit fließendem Blut,
- Schaffung eines Lochs in der Kalkschale des Vogeleies mittels einer Locherzeugungseinheit,
- Suchen der sich im Ei ausbildenden Blutgefäße oder des Herzens mittels eines Visions-Systems und einer koaxialen oder lateralen Beleuchtung mit Licht im sichtbarem Wellenlängenbereich,
- Positionieren zumindest eines Blutgefäßes oder des Herzens in den Laserfokus mindestens einer Laserstrahlenquelle entweder durch Bewegen des Eies oder durch Bewegen eines den Laserfokus erzeugenden Objektivs,
- Bestrahlung des Blutgefäßes oder des Herzens mit mindestens einer eine Anregungswellenlänge emittierenden Laserstrahlenquelle,
- Registrieren der Rückstreustrahlung des bestrahlten Blutgefäßes oder des Herzens mittels mindestens eines Detektors, der mit mindestens einer mit einer nachgeschalteten Amplifikations- und Detektoreinheit verbundenen Auswerteeinheit in Verbindung steht, wobei während der Registrierung eine Bewegung des Blutgefäßes oder des Herzens aus dem Laserstrahl heraus durch Nachführung der Blutgefäße oder des Herzens oder des Objektivs erfolgen kann,
wobei gemäß dem Kennzeichenteil des Patentanspruchs 1
folgende weitere Schritte folgen
- Auswertung der Rückstreustrahlung einschließlich der Fluoreszenzstrahlung in der Auswerteeinheit aus der registrierten spektralen Intensität der Fluoreszenzstrahlung in einem zur Anregungswellenlänge rotverschobenen Spektralbereich, wobei die geschlechtsspezifischen Eigenschaften des männlichen Blutes und des weiblichen Blutes in der Intensität und im Spektralprofil der registrierten Fluoreszenzstrahlung enthalten sind und wobei zumindest eine der aus den gemessenen spektralen Intensitäten der Fluoreszenzstrahlung ermittelten Intensitätsgrößen oder deren zugeordnete Größen bezüglich des männlichen Blutes gegenüber zumindest einer der ermittelten Intensitätsgrößen oder deren zugeordnete Größen bezüglich des weiblichen Blutes in den Blutgefäßen einen auswertbaren unterschiedlichen Wert aufweisen,
- Bestimmung des Geschlechts des Vogeleies aus dem Unterschied zumindest eines der Werte der Fluoreszenzintensitätsgrößen oder deren zugeordnete Größen in der Auswerteeinheit und danach zumindest
- eine Anzeige des in der Auswerteeinheit bestimmten Geschlechts des Embryos im Vogelei.

Der jeweils auswertbare unterschiedliche Wert der Intensitätsgrößen kann sich zumindest auf einen vorgegebenen, in der Auswerteinheit gespeicherten, der Intensitätsgröße zugeordneten Grenzwert oder Schwellwert beziehen.

In der Auswerteeinheit können die aus den ermittelten Fluoreszenzintensitäts/Wellenzahl-Kurven ermittelten Intensitätsgrößen bezüglich eines vorgegebenen Spektralbereiches als die integrale Intensität I_{I51} der Fluoreszenzstrahlung des männlichen Blutes und als die integrale Intensität I_{I52} der Fluoreszenzstrahlung des weiblichen Blutes festgelegt werden.

In der Auswerteeinheit können die aus den ermittelten Fluoreszenzintensitäts/Wellenzahl-Kurven ermittelten Intensitätsgrößen bezüglich eines vorgegebenen Spektralbereiches als das Intensitätsmaximum 51_{Max} der Fluoreszenzstrahlung des männlichen Blutes und als das Intensitätsmaximum 52_{Max} der Fluoreszenzstrahlung des weiblichen Blutes festgelegt werden.

In der Auswerteeinheit können bei Einsatz einer Laserstrahlenquelle die aus der ermittelten Fluoreszenzintensitäts/Wellenzahl-Kurve ermittelten Intensitätsgrößen bezüglich eines vorgegebenen Spektralbereiches jeweils für das männliche Blut und für das weibliche Blut mittels logischer Verknüpfung kombiniert zur Auswertung festgelegt werden.

In der Auswerteeinheit können bei Einsatz mindestes zweier Laserstrahlenquellen die aus mehreren ermittelten Fluoreszenzintensitäts/Wellenzahl-Kurven ermittelten Intensitätsgrößen bezüglich eines vorgegebenen Spektralbereiches als die integralen Intensitäten I_{I511}, I_{I512} der Fluoreszenzstrahlungen des männlichen Blutes und als die integralen Intensitäten I_{I521}, I_{I522} der Fluoreszenzstrahlungen des weiblichen Blutes festgelegt werden.

In der Auswerteeinheit können bei Einsatz mindestes zweier Laserstrahlenquellen die aus mehreren ermittelten Fluoreszenzintensitäts/Wellenzahl-Kurven ermittelten Intensitätsgrößen bezüglich eines vorgegebenen Spektralbereiches jeweils für das männliche Blut und für das weibliche Blut kombiniert zur Auswertung festgelegt werden.

In der Auswerteeinheit können die aus den ermittelten Fluoreszenzintensitäts/Wellenzahl-Kurven ermittelten Intensitätsgrößen bezüglich eines vorgegebenen Spektralbereiches als die integrale Intensität I_{I51} der Fluoreszenzstrahlung des männlichen Blutes und als die integrale Intensität I_{I52} der Fluoreszenzstrahlung des weiblichen Blutes und die jeweils die Fluoreszenzstrahlungen überlagernden Ramanstreustrahlungen 50₅₁, 50₅₂ für die Auswertung allein oder in Kombination mit den anderen festgelegten Intensitätsgrößen und zugeordneten Größen festgelegt werden, wobei bei Kombination der Intensitätsgrößen und zugeordneter Größen die Ramanstreustrahlung und die Fluoreszenzstrahlung mittels logischer Verknüpfung gemeinsam ausgewertet werden.

Bei einem Einsatz einer gepulsten Laserstrahlung aus der Laserstrahlenquelle kann die erzeugte Fluoreszenzintensität zeitaufgelöst gemessen und aus der Zeitkonstanten r der Abklingkurve der zeitaufgelösten Fluoreszenzintensität eine Geschlechtsbestimmung durchgeführt werden, wobei aus der Fluoreszenzabklingkurve für männliches Blut und für weibliches Blut jeweils eine unterschiedliche Zeitkonstante *τ*_{männlich}, *τ*_{weibllich} mit *τ*_{männlich} ≠ *τ*_{weibllich} zur Geschlechtsbestimmung ermittelt werden.

Unter Berücksichtigung der ebenfalls auftretenden und registrierten Ramanstreustrahlung können in der Auswerteeinheit die aus den ermittelten Fluoreszenzintensitäts/Wellenzahl-Kurven ermittelten Intensitätsgrößen bezüglich eines vorgegebenen Spektralbereiches als die integrale Intensität I_{I51} der Fluoreszenzstrahlung des männlichen Blutes und als die integrale Intensität I_{I52} der Fluoreszenzstrahlung des weiblichen Blutes und die jeweils die Fluoreszenzstrahlungen überlagernden Ramanstreustrahlungen 50₅₁, 50₅₂ für die Auswertung festgelegt werden.

Eine Vorrichtung zur optischen in-ovo Geschlechtsbestimmung eines befruchteten und bebrüteten Vogeleies auf der Basis entstehender Fluoreszenzstrahlung umfasst
- eine Ei-Lagerungseinheit, auf der das Ei gelagert ist,
- eine Positions-Auswerteeinrichtung, die mit der Ei-Lagerungseinheit in Verbindung steht,
- eine Strahlungseinrichtung mit Licht im sichtbaren Wellenbereich zur Bestrahlung zumindest eines extraembryonalen Blutgefäßes, eines embryonalen Blutgefäßes oder des Herzens des Embryos,
- einen Detektor für sichtbares oder grünes Licht zur Erkennung zumindest eines extraembryonalen Blutgefäßes, eines embryonalen Blutgefäßes oder des Herzens des Embryos, wobei der Detektor mit der Positionier-Auswerteeinrichtung in Verbindung steht,
- eine Vorrichtung zur Einbringung von Laserlicht in das Ei, die zumindest umfasst:
- eine Laserlicht emittierenden Laserquelle,
- einen Detektor zur Aufnahme der Fluoreszenzstrahlung,
- eine Steuereinheit zu xyz-Positionierung der Vorrichtung auf das in das Ei eingebrachte Loch,
- eine Geschlechtsbestimmungs-Auswerteeinheit, die mit der Amplifikations- und Detektioneinheit und der Positionier-Auswerteeinrichtung, die mit der Steuereinheit verbunden ist, in Verbindung steht,
wobei gemäß dem Kennzeichenteil des Patentanspruchs 14 eine Vorrichtung zur Separation der Fluoreszenzstrahlung aus der Rückstreustrahlung zwischen einem Kollimator für die Strahlbündelung des Laserstrahls und der Vorrichtung zur Einbringung des Laserstrahls in das Ei angeordnet ist, wobei zwischen der strahlseparierenden Vorrichtung und mindestens einem Fluoreszenz-Detektor jeweils ein strahlengangzugehöriger Detektionsfilter für die Transmission der Fluoreszenzstrahlung eingebracht ist,
wobei in der Geschlechtsbestimmungs-Auswerteeinheit erfolgen
- eine Auswertung der Rückstreustrahlung einschließlich der Fluoreszenzstrahlung aus der registrierten spektralen Intensität der Fluoreszenzstrahlung in einem zur Anregungswellenlänge rotverschobenen Spektralbereich, wobei die geschlechtsspezifischen Eigenschaften des männlichen Blutes und des weiblichen Blutes in der Intensität und im Spektralprofil der registrierten Fluoreszenzstrahlung enthalten sind und wobei zumindest eine der aus den gemessenen spektralen Intensitäten der Fluoreszenzstrahlung ermittelten Intensitätsgrößen oder deren zugeordnete Größen bezüglich des männlichen Blutes gegenüber zumindest einer der ermittelten Intensitätsgrößen oder deren zugeordnete Größen bezüglich des weiblichen Blutes in den Blutgefäßen einen auswertbaren unterschiedlichen Wert aufweisen,
- eine Bestimmung des Geschlechts des Vogeleies aus dem Unterschied zumindest eines der Werte der Fluoreszenzintensitätsgrößen oder deren zugeordnete Größen in der Auswerteeinheit.

Das Licht im sichtbaren Wellenlängenbereich, mit dem die Strahlungseinrichtung zur Bestrahlung zumindest eines Blutgefäßes vorgesehen ist, stellt vorzugsweise grünes Licht dar.

Der Laserstrahl, der im Laser produziert wird, kann mittels Spiegeln oder mittels Faseroptik übertragen werden. Der Laserstrahl wird mit dem Kollimator gebündelt.

Der Laserstrahl wird zum Ei übertragen und mit einer Linse auf ein extraembryonales Blutgefäß, ein embryonales Blutgefäß oder das Herz des Embryos fokussiert.

Die entstehende Fluoreszenzstrahlung wird mit der gleichen Linse gesammelt.

Ein Strahlteiler wird genutzt, um das Fluoreszenzsignal vom sichtbaren Licht der Strahlungsquelle zu separieren.

Das Licht im sichtbaren Spektralbereich wird zur Kamera übertragen, ein Filter entfernt verbleibende Strahlen des Laserlichtes.

Ein Strahlteiler wird genutzt, um das Fluoreszenzlicht zu separieren, welches auf den Detektor auftrifft. Der Detektor, typischerweise eine Photodiode, vorzugsweise eine Lawinenphotodiode eines Photomultiplier, misst die Intensität der Fluoreszenzstrahlung.

Mindestens ein Bandpassfilter selektiert den zu messenden Spektralbereich der Fluoreszenz.

Eine Detektionseinheit verstärkt, filtert und misst das Signal des Detektors, welches von ihr aus zur Geschlechtsbestimmungseinheit gesendet wird.

Die gemessene Intensität wird in der Auswerteeinheit ausgewertet, beispielsweise durch einen Vergleich mit einem gemessenen Schwellwert SW, und damit ist das Geschlecht des Eies bestimmt.

Das Einbringen eines Loches in die Kalkschale mittels einer Locherzeugungseinheit in Form eines Lasers oder mechanischer Perforation kann mit Durchmessern mit bis zu 18 mm, vorzugsweise zwischen 4 mm und 15 mm durchgeführt werden. Das Licht im sichtbaren Wellenlängenbereich kann weißes Licht sein, jedoch wird der Kontrast verbessert bei Einsatz von blauem und/oder grünem Licht aus einer Lichtquelle (z.B. grüner oder blauer LED, oder Glühlampe mit blauem oder grünem Filter). Während der Messung kann eine Bewegung des Blutgefäßes oder des Herzens aus dem Laserstrahl heraus durch Nachführung der Blutgefäße oder des Herzens oder des zugehörigen Objektivs, ausgelöst durch das überwachende Visions-System, erfolgen.

Fluoreszenzstrahlungsmessungen werden unter Nutzung von optischen Systemen wie Linsen oder Mikroskop-Objektiven oder einer Fasersonde durchgeführt.

Der fokussierte oder gebündelte Laserstrahl wird auf das ausgewählte Blutgefäß oder auf das Herz fokussiert und die Nachführung bei Bewegung des ausgewählten Blutgefäßes oder des Herzens mittels einer Linse oder eines Objektivs gegebenenfalls automatisch durchgeführt.

Die Anregungswellenlängen des Laserlichts der Laserstrahlenquelle sind größer als 400 nm, unter Verwendung z.B. HeNe-Laser 633 nm, Festkörperlaser (Nd basiert z.B. Nd:YAG Laser 532nm oder 1064nm, VIS NIR Diodenlaser z.B. 785 nm). Die Einkopplung des Laseranregungsstrahles kann direkt mit Spiegeln und/oder mit optischen Fasern erfolgen. Die eingebrachte Leistung der Laserstrahlenquelle darf nicht zu einer lokalen und globalen Erwärmung des Eies über 40° Celsius führen.

Eine direkte Auskopplung der gesammelten Rückstreustrahlung erfolgt mit Spiegeln oder mit optischen Fasern bis zur Detektion. Die Rückstreustrahlung enthält als einen Teil die Fluoreszenzstrahlung.

Die Fluoreszenzstrahlung kann vorzugsweise mit einem Objektiv registriert werden.

Das jeweilige optische Element zur Strahlführung oder zur rückführenden Strahlführung kann eine flexible optische Faser sein.

Eine Detektion der Rückstreustrahlung kann folgendermaßen durchgeführt werden, durch:
- Anwendung von Geräten, welche die Lichtintensität oder den Photonenfluss messen, wie z.B. Photodioden, Lawinenphotodioden oder Photomultiplier,
- Anwendung von Strahlteilern und optischen Bandpassfiltern vor der Detektion, um die spektralen Bereiche der Fluoreszenz zu selektieren, oder
- Anwendung von dispersiven Spektrometern, um eine spektrale Analyse durchzuführen.

Die geschlechtsspezifischen Merkmale sind in der Intensität und im Spektralprofil der Fluoreszenz enthalten, wobei die ermittelten Fluoreszenzintensitäten in ein oder mehreren Spektralbereichen einer mathematischen Analyse zugeführt werden.

Die Geschlechtsbestimmung kann an jedem Tag zwischen der Ausbildung mindestens eines erkennbaren Blutgefäßes oder der Ausbildung des Herzens und dem Schlupf vorgenommen werden, vorzugsweise etwa am Tag 2,5 bis Tag 5 der Bebrütung. Nach dem fünften Bruttag nimmt der Unterschied zwischen der Autofluoreszenz des Blutes von männlichen Embryonen und weiblichen Embryonen ab.

Die Vorteile der erfindungsgemäßen Geschlechtsbestimmung sind:
- keine Beeinträchtigung des Schlupfes und der nachfolgenden Entwicklung des Kükens und
- Durchführung der Geschlechtsbestimmung mit hoher Genauigkeit in Echtzeit zu einem sehr frühen Zeitpunkt und mithilfe einer kontaktlosen Bestimmung ohne Probenentnahme.

Die registrierte spektrale Rückstreustrahlung ist variabel im Spektralbereich, aber immer rotverschoben im Vergleich zur Anregungswellenlänge des Lasers. Typisch ist eine Frequenzverschiebung zwischen ca. 100 cm⁻¹ and 4000cm⁻¹. In dem Spektralbereich ist die Autofluoreszenz des Blutes für männliche Embryonen und für weibliche Embryonen unterschiedlich. Die Intensität der Autofluoreszenz des männlichen Blutes ist stärker und das spektrale Fluoreszenz-Maximum der Intensität des männlichen Blutes ist leicht zu höheren Wellenzahlen verschoben. Die schwache Ramanstrahlung ist der Fluoreszenzstrahlung überlagert.

Im Folgenden werden vier verschiedene Konfigurationen der erfindungsgemäßen Vorrichtung aufgezeigt. Die Konfigurationen sind für die Anwendung an einem Ei beschrieben, Parallelisierungen der erfindungsgemäßen Vorrichtung, d.h. eine Anordnung von mehreren erfindungsgemäßen Vorrichtungen in einer Geschlechtsbestimmungs-Strecke sind möglich, um dann automatisch eine höhere Ei-Anzahl in gleicher Zeit bezüglich des Geschlechts analysieren zu können.

Weiterbildungen und vorteilhafte Ausgestaltungen der Erfindung sind in weiteren Unteransprüchen angegeben.

Die Erfindung wird mittels Ausführungsbeispielen anhand von Zeichnungen näher erläutert.

Es zeigen:

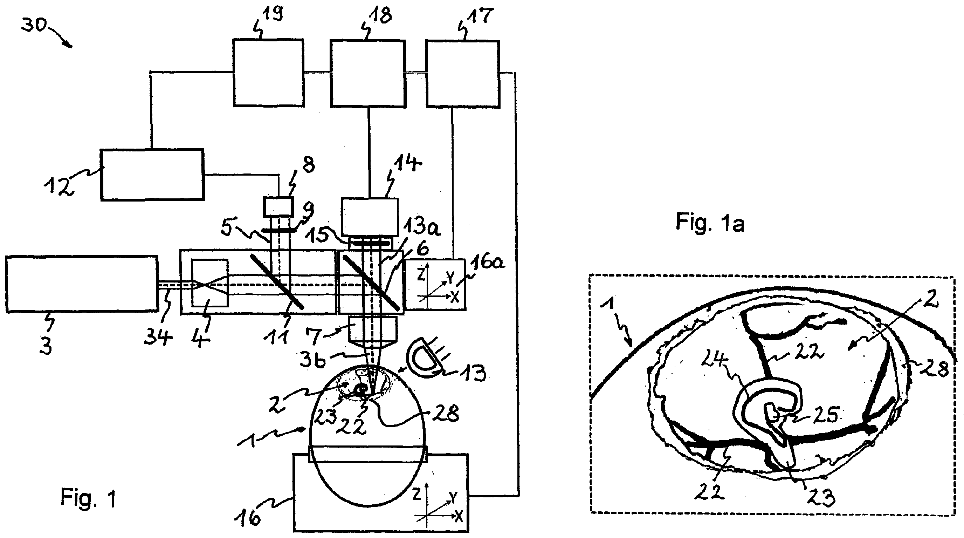

- Fig. 1: eine schematische Darstellung einer erfindungsgemäßen ersten Vorrichtung zur Bestimmung des Geschlechtes von bebrüteten Vogeleiern auf der Basis entstehender Fluoreszenzstrahlung nach Anregung mit Messung der integralen Intensität für das männliche Geschlecht und für das weibliche Geschlecht des Embryos mit einer Vorrichtung zur Separation der Fluoreszenzstrahlung und einem Detektor, wobei Fig. 1a einen Einblick in die Lochöffnung mit dem Embryo, den zugehörigen Blutgefäßen und den extraembryonalen blutführenden Gefäßen gemäß Fig. 1 vergrößert darstellt,

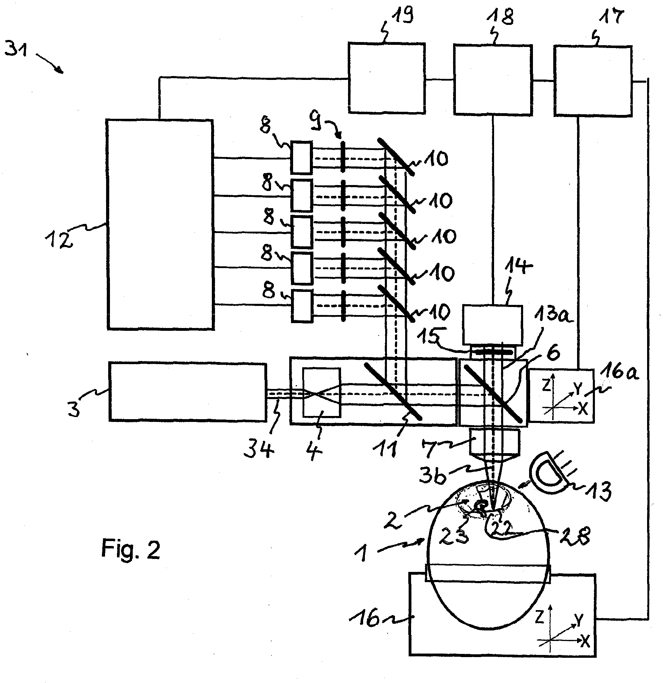

- Fig. 2: eine schematische Darstellung einer erfindungsgemäßen zweiten Vorrichtung zur Bestimmung des Geschlechtes von bebrüteten Vogeleiern auf der Basis entstehender Fluoreszenzstrahlung nach Anregung mit Messung bei vorgegebenen Wellenzahlen mittels einer Vorrichtung zur Separation der Fluoreszenzstrahlung und einer Anzahl von Detektoren,

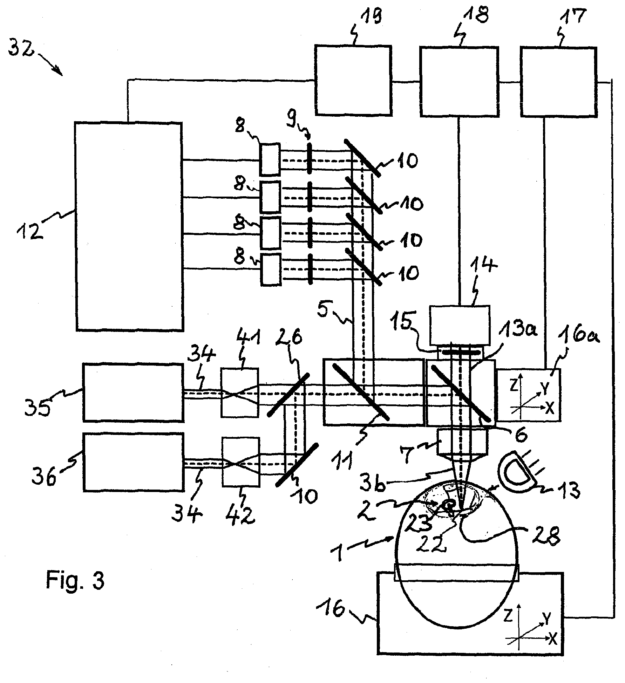

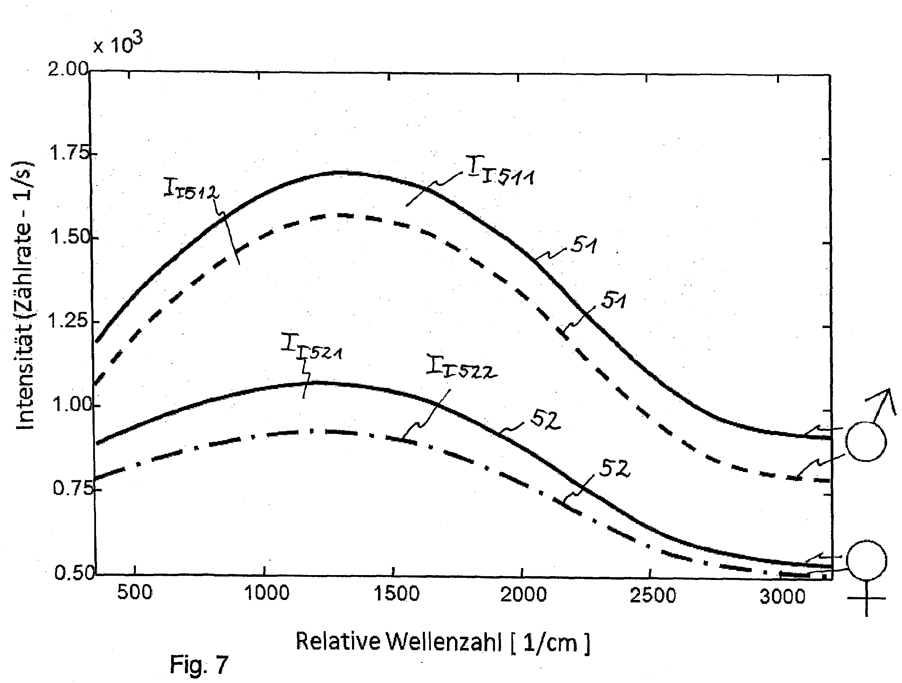

- Fig. 3: eine schematische Darstellung einer erfindungsgemäßen dritten Vorrichtung zur Bestimmung des Geschlechtes von bebrüteten Vogeleiern auf der Basis entstehender Fluoreszenzstrahlung nach Anregung mit mindestens zwei Laserquellen und der Messung der jeweils beiden in Fig. 7 als integrale Intensitäten dargestellten Kurven für das männliche Geschlecht und für das weibliche Geschlecht des Embryos bei vorgegebenen Wellenzahlen mittels einer Vorrichtung zur Separation der Fluoreszenzstrahlung und mehreren Detektoren,

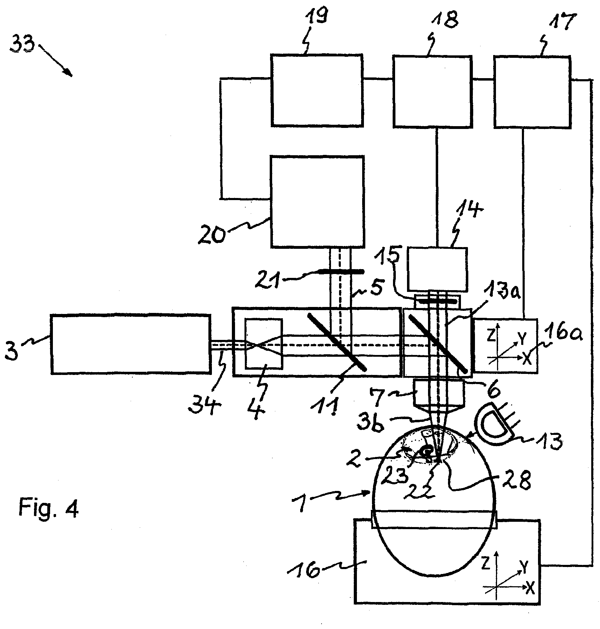

- Fig. 4: eine schematische Darstellung einer erfindungsgemäßen vierten Vorrichtung zur Bestimmung des Geschlechtes von bebrüteten Vogeleiern auf der Basis entstehender Fluoreszenzstrahlung und der Ramanstreustrahlung nach Anregung mit den Messungen der beiden Intensitätsverläufe mittels einer Vorrichtung zur Separation der Fluoreszenzstrahlung und einem Spektrometer,

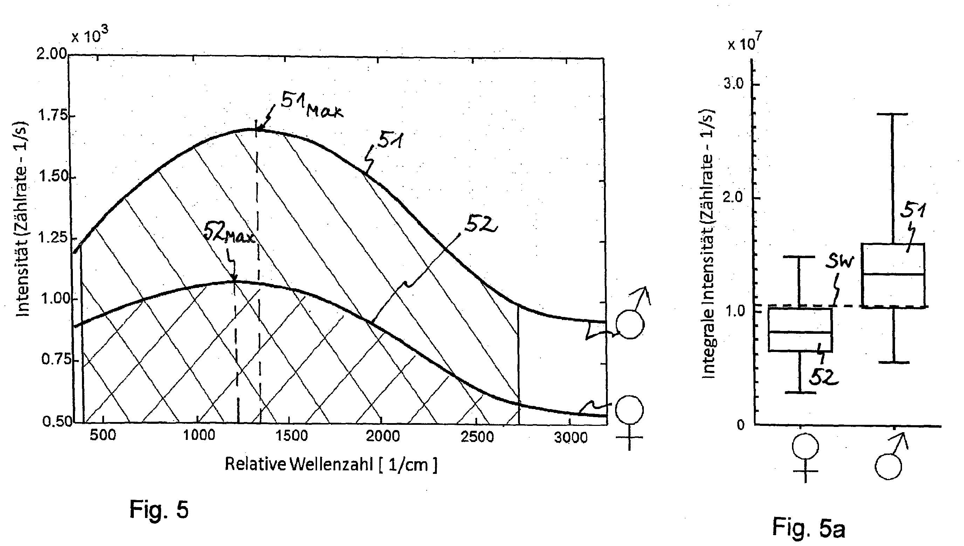

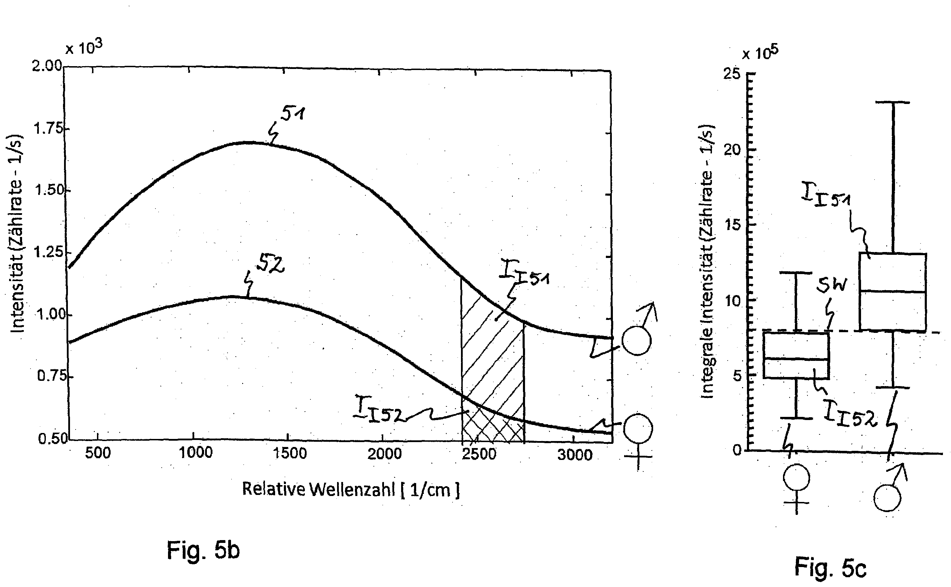

- Fig. 5: Fluoreszenzintensitäts/Wellenzahl-Kurven für die erfindungsgemäße erste Vorrichtung nach Fig. 1 zur Bestimmung des Geschlechtes von bebrüteten Vogeleiern auf der Basis entstehender Fluoreszenzstrahlung nach Anregung mit Messung der integralen Intensität (jeweils unterschiedlich schraffiert), wobei Fig. 5a die integralen Intensitäten nach Fig. 5 für den gesamten Spektralbereich, Fig. 5b den spektralen Intensitätsverlauf für einen vorgegebenen, anteiligen Spektralbereich und Fig. 5c die zu Fig. 5b zugehörigen integralen Intensitäten, zeigen,

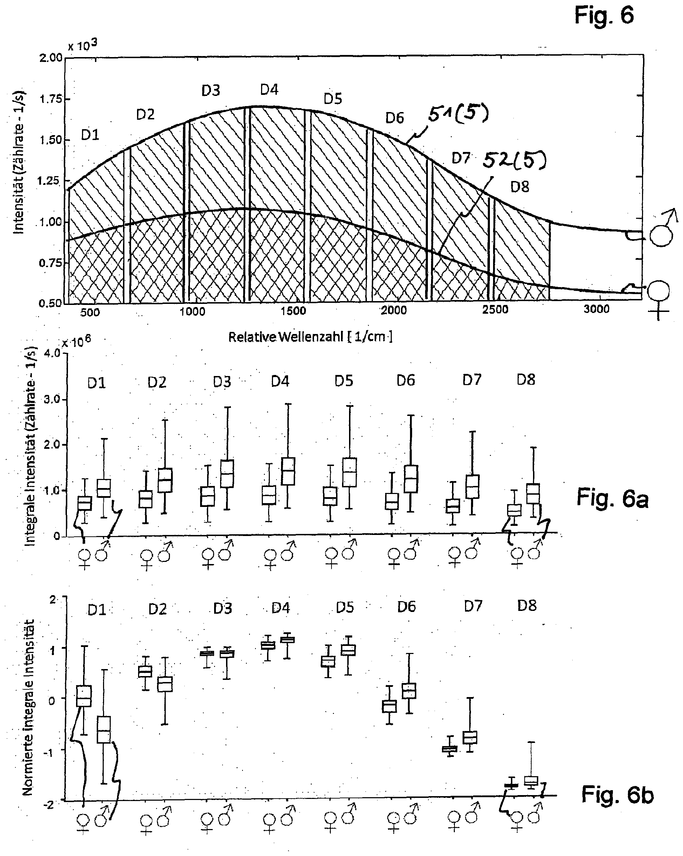

- Fig. 6: Fluoreszenzintensitäts/Wellenzahl-Kurven für die erfindungsgemäße zweite Vorrichtung nach Fig. 2 zur Bestimmung des Geschlechtes von bebrüteten Vogeleiern auf der Basis entstehender Fluoreszenzstrahlung nach Anregung mit Messung der Intensität an den registrierten Intensitätskurven bei jeweils vorgegebenen Wellenzahlen, wobei Fig. 6a die integralen Intensitäten von festgelegten teilweisen Spektralbereichen über den gesamten Spektralbereich und Fig. 6b die normierte integrale Intensität nach Fig. 6 und Fig. 6a zeigen,
- Fig. 7: Fluoreszenzintensitäts/Frequenzzahl-Kurven für die erfindungsgemäße dritte Vorrichtung nach Fig. 3 zur Bestimmung des Geschlechtes von bebrüteten Vogeleiern auf der Basis entstehender Fluoreszenzstrahlung nach Anregung mittels zwei unterschiedliche Wellenlängen emittierenden Laserstrahlenquellen mit Messung der unterschiedlichen Intensitätskurven (jeweils durchgezogen, gestrichelt),

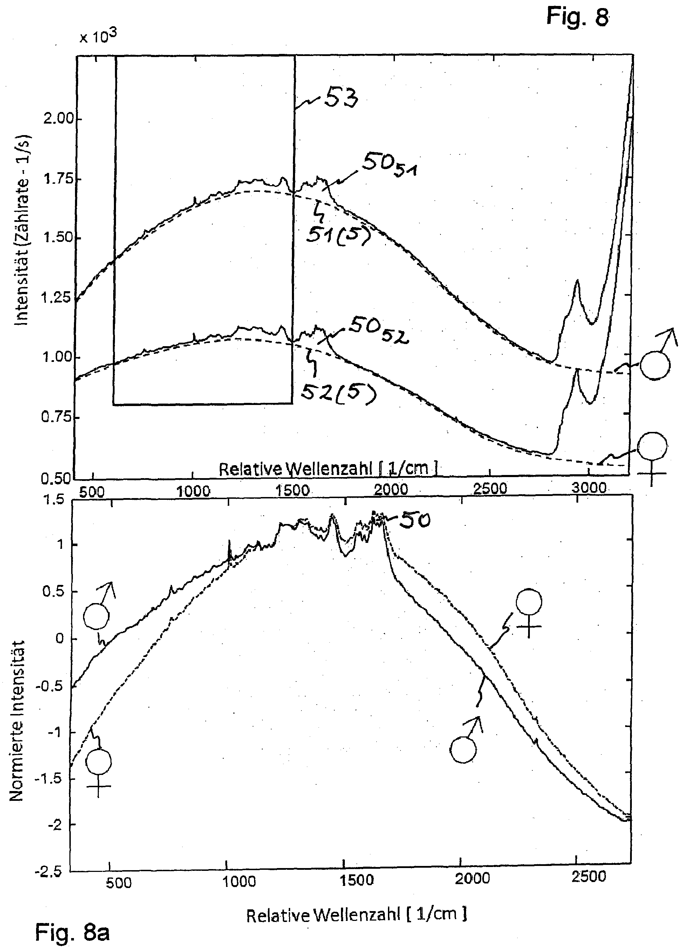

- Fig. 8: Fluoreszenzintensitäts/Frequenzzahl-Kurven für die erfindungsgemäße vierte Vorrichtung nach Fig. 4 zur Bestimmung des Geschlechtes von bebrüteten Vogeleiern auf der Basis entstehender Fluoreszenzstrahlung und Ramanstreustrahlung nach Anregung mit einer Laserstrahlenquelle zur Messung der beiden sich überlagernden Intensitäten, wobei Fig. 8a die Kurven der normierten Intensitäten nach Fig. 8 zeigt.

In Fig. 1 ist eine schematische Darstellung einer ersten Vorrichtung 30 zur optischen in-ovo Geschlechtsbestimmung eines befruchteten und bebrüteten Vogeleies 1 auf der Basis entstehender Fluoreszenzstrahlung 5 gezeigt, wobei die Vorrichtung 30 umfasst
- eine Ei-Lagerungseinheit 16, auf der das Ei 1 gelagert ist,
- eine Positions-Auswerteeinrichtung 17, die mit der Ei-Lagerungseinheit 16 in Verbindung steht,
- eine Strahlungseinrichtung 13 mit Licht im sichtbaren Wellenbereich zur Bestrahlung zumindest eines extraembryonalen Blutgefäßes 22, eines embryonalen Blutgefäßes 24 oder des Herzens 25 des Embryos 23,
- einen Detektor (z.B. eine Kamera) 14 für sichtbares oder grünes Licht 13a zur Erkennung zumindest eines extraembryonalen Blutgefäßes 22, eines embryonalen Blutgefäßes 24 oder des Herzens 25 des Embryos 23, wobei der Detektor 14 mit der Positionier-Auswerteeinrichtung 18 in Verbindung steht,
- eine Vorrichtung 6 zur Einbringung von Laserlicht 34 in das Ei 1,
die zumindest in Verbindung steht mit
- einer Laserlicht 34 emittierenden Laserquelle 3,
- einem Detektor 8 zur Aufnahme der Fluoreszenzstrahlung 5,
- einer Steuereinheit 17 zu xyz-Positionierung der Vorrichtung 6 auf das in das Ei 1 eingebrachte Loch 2,
- einer Geschlechtsbestimmungs-Auswerteeinheit 19, die mit der Amplifikations- und Detektioneinheit 12 und der Positionier-Auswerteeinrichtung 18, die mit der Steuereinheit 17 verbunden ist, in Verbindung steht.

In Fig. 1 ist erfindungsgemäß eine Vorrichtung 11 zur Separation der Fluoreszenzstrahlung 5 aus der Rückstreustrahlung zwischen einem Kollimator 4 für die Strahlbündelung des Laserstrahls 34 und der Vorrichtung zur Einbringung des Laserlichts 34in das Ei 1 angeordnet, wobei zwischen der strahlseparierenden Vorrichtung 11 und mindestens einem Fluoreszenz-Detektor 8 jeweils ein strahlengangzugehöriger Detektionsfilter 9 für die Transmission der Fluoreszenzstrahlung 5 eingebracht ist.

Der Detektionsfilter 9 kann ein angepasster vorgegebener Bandpassfilter sein.

In der Geschlechtsbestimmungs-Auswerteeinheit 19 können erfolgen
- eine Auswertung der Rückstreustrahlung 5, 50, 51, 52 einschließlich der Fluoreszenzstrahlung 5, 51, 52 aus der registrierten spektralen Intensität der Fluoreszenzstrahlung 51, 52 in einem zur Anregungswellenlänge rotverschobenen Spektralbereich, wobei die geschlechtsspezifischen Eigenschaften des männlichen Blutes und des weiblichen Blutes in der Intensität und im Spektralprofil der registrierten Fluoreszenzstrahlung 51, 52 enthalten sind und wobei zumindest eine der aus den gemessenen spektralen Intensitäten der Fluoreszenzstrahlung 51, 52 ermittelten Intensitätsgrößen oder deren zugeordnete Größen bezüglich des männlichen Blutes gegenüber zumindest einer der ermittelten Intensitätsgrößen oder deren zugeordnete Größen bezüglich des weiblichen Blutes in den Blutgefäßen 22, 24, 25 zumindest einen auswertbaren unterschiedlichen Wert aufweisen, und somit
- eine Bestimmung des Geschlechts des Vogeleies 1 aus dem Unterschied zumindest eines der Werte der Fluoreszenzintensitätsgrößen 51, 52 oder deren zugeordnete Größen oder aus einem Vergleich mit einem Schwellwert SW mittels programmtechnischen Mitteln und/oder optionalen, in der Auswerteeinheit 19 vorhandenen logischen Verknüpfungen
   erfolgen.

Das Licht im sichtbaren Wellenlängenbereich, mit dem die Strahlungseinrichtung 13 zur Bestrahlung zumindest eines Blutgefäßes 22, 24 oder des Herzens 25 vorgesehen ist, kann vorzugsweise grünes Licht darstellen.

Der Laserstrahl, der im Laser 3 produziert wird, kann mittels Spiegeln oder mittels Faseroptik übertragen werden. Der Laserstrahl 34 wird mit dem Kollimator 4 gebündelt.

Der Laserstrahl 34 wird zum Ei übertragen und mit einer Linse 7 auf ein extraembryonales Blutgefäß 22, ein embryonales Blutgefäß 24 oder das Herz 25 des Embryos 23 fokussiert.

Die entstehende Fluoreszenzstrahlung 5 wird mit der gleichen Linse 7 gesammelt.

Ein Strahlteiler 6 wird genutzt, um das Fluoreszenzsignal 5 vom sichtbaren Licht 13a der Strahlungsquelle 13 zu separieren.

Das Licht im sichtbaren Spektralbereich 13a wird zur Kamera 14 übertragen, ein Filter 15 entfernt verbleibende Strahlen des Laserlichtes.

Ein Strahlteiler 11 wird genutzt, um die Fluoreszenzstrahlung 5 zu separieren, welches auf den Detektor 8 auftrifft. Der Detektor 8, typischerweise eine Photodiode oder eine Lawinenphotodiode oder ein Photomultiplier, misst die Intensität der Fluoreszenzstrahlung.

Der Bandpassfilter 9 selektiert den zu messenden Spektralbereich der Fluoreszenz.

Eine Detektionseinheit 12 verstärkt, filtert und misst das Signal des Detektors, welches von ihr aus zur Geschlechtsbestimmungs-Auswerteeinheit 19 gesendet wird.

Die gemessene Intensität kann mit einem gemessenen Schwellwert in der Auswerteeinheit 19 verglichen werden, und damit ist das Geschlecht des Eies bestimmt.

Die Fig. 1a stellt eine Vergrößerung des Loches 2 gemäß Fig. 1 dar. Darin sind extraembryonale Blutgefäße 22, embryonale Blutgefäße 24 und der Embryo 23 mit dem darin befindlichen Herz 25 vorhanden.

Die auf die erste Vorrichtung 30 bezogene, in Fig. 5 dargestellte, registrierte spektrale Strahlung ist variabel im Spektralbereich, aber immer rotverschoben im Vergleich zur Anregungswellenlänge des Lasers 3. Typisch ist eine Frequenzverschiebung zwischen 100 cm⁻¹ and 4000 cm⁻¹. In dem in Fig. 5 angegebenen rotverschobenen Spektralbereich ist die Autofluoreszenz des Blutes für männliche Embryonen und für weibliche Embryonen unterschiedlich. Die Autofluoreszenz des männlichen Blutes weist einen höheren Intensitätsverlauf 51 als der Intensitätsverlauf 52 bezüglich des weiblichen Blutes auf und das spektrale Fluoreszenz-Maximum 51_{Max} des männlichen Blutes ist gegenüber dem Fluoreszenz-Maximum 52_{Max} des weiblichen Blutes leicht zu höheren Wellenzahlen verschoben.

In der Fig. 5 sind die Fluoreszenzintensitäts/Frequenzzahl-Kurven für die erfindungsgemäße erste Vorrichtung 30 nach Fig. 1 zur Bestimmung des Geschlechtes von bebrüteten Vogeleiern auf der Basis entstehender Fluoreszenzstrahlung nach Anregung mit Messung der integralen Intensität, wie in Fig. 5a gezeigt ist, (jeweils unterschiedlich schraffiert) gezeigt.

In der Auswerteeinheit 19 können gemäß Fig. 5, 5b und 5c zumindest die aus den ermittelten Fluoreszenzintensitäts/Wellenzahl-Kurven 51, 52 ermittelten Intensitätsgrößen bezüglich des vorgegebenen Spektralbereiches zwischen ca. 2400 und 2750 cm⁻¹als die integrale Intensität I_{I51} der Fluoreszenzstrahlung 51 des männlichen Blutes und als die integrale Intensität I_{I52} der Fluoreszenzstrahlung 52 des weiblichen Blutes gemäß Fig. 5c zur Bestimmung des Geschlechts festgelegt werden.

In der Konfiguration 1 gemäß Fig. 1 wird Folgendes durchgeführt:
- Die Anregung erfolgt mit einer Wellenlänge aus einem Laser 3.
- Der gesamte Spektralbereich oder ein spektraler Teilbereich der Fluoreszenzstrahlung und Rückstreustrahlung wird mit einem Detektor/Sensor 8 und mit vorgegebenem Bandpassfilter 9 registriert.
- Das Signal wird evtl. verstärkt.
- Für die registrierte und evtl. verstärkte Intensität ist ein angepasster Schwellwert SW festgelegt, um weibliche Embryonen und männliche Embryonen zu unterscheiden. Das Blut weiblicher Embryonen ist charakterisiert durch einen Intensitätswert I_{I52}, der geringer ist als der angepasste Schwellwert SW, das Blut männlicher Embryonen ist charakterisiert durch einen Intensitätswert I_{I51}, der größer ist als der angepasste Schwellwert SW.

Des Weiteren können gemäß Fig. 5 in der Auswerteeinheit 19 die aus den ermittelten Fluoreszenzintensitäts/Wellenzahl-Kurven 51, 52 ermittelten Intensitätsgrößen bezüglich eines vorgegebenen Spektralbereiches als das Intensitätsmaximum 51_{Max} der Fluoreszenzstrahlung 51 des männlichen Blutes und als das Intensitätsmaximum 52_{Max} der Fluoreszenzstrahlung 52 des weiblichen Blutes zur Bestimmung des Geschlechts festgelegt werden.

In der der Konfiguration 2 entsprechenden Fig. 2 ist in einer schematischen Darstellung eine zweite Vorrichtung 31 zur in-ovo Geschlechtsbestimmung eines befruchteten und bebrüteten Vogeleies 1 auf der Basis einer detektierten Fluoreszenzstrahlung 5 gezeigt, wobei die Vorrichtung 31 umfasst
- eine Ei-Lagerungseinheit 16, auf der das Ei 1 gelagert ist,
- eine Positions-Auswerteeinrichtung 17, die mit der Ei-Lagerungseinheit 16 in Verbindung steht,
- eine Strahlungseinrichtung 13 mit Licht im sichtbaren, vorzugsweise im grünen Wellenlängenbereich zur Bestrahlung zumindest eines Blut führenden Gefäßes, wie extraembryonalen Blutgefäßes 22, eines embryonalen Blutgefäßes 24 oder des Herzens 25 des Embryos 23,
- einen Detektor (z.B. Kamera) 14 für sichtbares oder grünes Licht 13a zur Erkennung zumindest eines extraembryonalen Blutgefäßes 22, eines embryonalen Blutgefäßes 24 oder des Herzens 25 des Embryos 23, wobei der Detektor 14 mit der Positionier-Auswerteeinrichtung 18 in Verbindung steht,
- eine Vorrichtung 6 zur Einbringung von Laserlicht 34 in das Ei 1,
die zumindest in Verbindung steht mit
- einer Laserlicht 34 emittierenden Laserquelle 3 und
- zwei oder mehreren/in Fig. 2 fünf bzw. in der Fig. 6 entsprechend acht vorgestellte Detektoren 8 zur Aufnahme der Fluoreszenzstrahlung 5 in zwei oder mehreren spektralen Bereichen D1 bis D8, die selektiert werden mittels vorgegebenen/in Fig. 2 fünf bzw. in der Fig. 6 entsprechend vorgestellten acht (nur fünf gezeichnet) Bandpassfiltern 9 und
- einer Steuereinheit 17 zu xyz-Positionierung der Vorrichtung 6 auf das in das Ei 1 eingebrachte Loch 2,
- eine Geschlechtsbestimmungs-Auswerteeinheit 19, die mit der Amplifikations- und Detektioneinheit 12 und der Positionier-Auswerteeinrichtung 18, die mit der Steuereinheit 17 verbunden ist, in Verbindung steht.

In der Konfiguration 2 gemäß Fig. 2 wird Folgendes durchgeführt:
- Die Anregung erfolgt mit einer Wellenlänge (Laserstrahlenquelle 3).
- Der gesamte Spektralbereich oder ein spektraler Teilbereich der Fluoreszenz und Rückstreuung wird mit mehr als einem Detektor/Sensor 8 und vorgegebenen Bandpassfiltern 9, welche verschiedene Spektralbereiche D1 bis D8 selektieren, registriert.
- Das Signal wird evtl. verstärkt.
- Um das Geschlecht des Embryos 23 zu erhalten, werden die registrierten und evtl. verstärkten Signalintensitäten einer mathematischen Auswertung zugeführt. Die mathematische Auswertung kann auf der Evaluation von Intensitäten, Intensitätsverhältnissen, -summen oder -differenzen basieren als auch überwachte Klassifikationsmethoden nutzen wie die Diskriminanzanalyse, Support-Vektor-Maschinen oder neuronale Netzwerke. Die registrierten Signalintensitäten können ohne Datenvorbehandlung ausgewertet werden oder vor der Evaluation normiert werden, beispielsweise durch Vektor- oder Flächennormierung.

Dazu können gemäß Fig. 6 in der Auswerteinheit 19 die aus den ermittelten Fluorezenzintensitäts/Wellenzahl-Kurven 51, 52 ermittelten Intensitätsgrößen I_{D1} bis I_{D8} bezüglich der acht vorgegebenen Spektralbereiche D1 bis D8 als integrale Intensitäten der Fluoreszenzstrahlung 51 des männlichen Blutes und als integrale Intensitäten der Fluoreszenzstrahlung 52 des weiblichen Blutes, bei der z.B. die Differenz der jeweiligen integralen Intensitäten am größten ist, zur Bestimmung des Geschlechts festgelegt werden.

Der in Fig. 2 dargestellte Laserstrahl, der im Laser 3 produziert wird, kann mittels Spiegeln oder mittels Faseroptik übertragen werden. Der Laserstrahl 34 wird mit dem Kollimator 4 gebündelt.

Der Laserstrahl 34 wird zum Ei übertragen und mit einer Linse oder Objektiv 7 auf ein extraembryonales Blutgefäß 22, ein embryonales Blutgefäß 24 oder das Herz 25 des Embryos 23 fokussiert.

Die Fluoreszenzstrahlung 5 wird mit derselben Linse/demselben Objektiv 7 gesammelt.

Ein Strahlteiler 6 wird genutzt, um das Fluoreszenzsignal 5 vom sichtbaren Licht 13a der Strahlungsquelle 13 zu separieren.

Das Licht im sichtbaren Spektralbereich 13a wird zur Kamera 14 übertragen, ein Filter 15 entfernt verbleibende Strahlen des Laserlichtes.

Der Strahlteiler 11 als Vorrichtung zum Trennen der Fluoreszenzstrahlung wird genutzt, um die Fluoreszenzstrahlung 5 zu separieren, welches auf den Detektor 8 auftrifft. Ein Detektor 8, typischerweise eine Photodiode oder eine Lawinenphotodiode oder ein Photomultiplier, misst die Intensität der Fluoreszenzstrahlung.

Die gemäß Fig. 6 vorgestellten acht (nur fünf gezeichnet) Bandpassfilter 9 selektieren die Spektralbereiche D1 bis D8 der Fluoreszenz, die mit jedem der vorgestellten acht (nur fünf eingezeichnet) Detektoren 8 registriert werden.

Eine Detektionseinheit 12 verstärkt, filtert und misst das Signal der Detektoren 8, welches zur Geschlechtsbestimmungs-Auswerteeinheit 19 gesendet wird.

Um das Geschlecht zu bestimmen, werden die Signalintensitäten mit einer der folgenden Verfahren oder einer Kombination der folgenden Verfahren in der Auswerteeinheit 19 ausgewertet:
- Intensitäten werden mit Schwellwerten verglichen,
- Intensitätsverhältnisse werden mit Schwellwerten verglichen,
- Intensitätssummen oder -differenzen werden mit Schwellwerten verglichen,
- Überwachte Klassifikationsmethoden wie beispielsweise Diskriminanzanalysen, Support-Vektor-Maschinen oder neuronale Netzwerke werden auf die Intensitätswerte angewendet.

Die Signalintensitäten I_{D1} bis I_{D8} können normiert werden, wie in Fig. 6b gezeigt ist, beispielsweise durch Vektor- oder Flächennormierung, und anschließend mathematisch mit einer der folgenden Verfahren oder einer Kombination der folgenden Verfahren ausgewertet, um das Geschlecht zu erhalten:
- Normierte Intensitäten in Fig. 6b werden z.B. mit Schwellwerten verglichen,
- Verhältnisse zwischen normierten Intensitäten werden mit Schwellwerten verglichen,
- Summen oder -differenzen normierter Intensitäten werden mit Schwellwerten verglichen,
- Überwachte Klassifikationsmethoden wie beispielsweise Diskriminanzanalysen, Support-Vektor-Maschinen oder neuronale Netzwerke werden auf die normierten Intensitätswerte angewendet.

In der der Konfiguration 3 entsprechenden Fig. 3 ist in einer schematischen Darstellung eine dritte Vorrichtung 32 zur optischen in-ovo Geschlechtsbestimmung eines befruchteten und bebrüteten Vogeleies 1 auf der Basis der Fluoreszenzstrahlung gezeigt, wobei die Vorrichtung 32 umfasst
- eine Ei-Lagerungseinheit 16, auf der das Ei 1 gelagert ist,
- eine Positions-Auswerteeinrichtung 17, die mit der Ei-Lagerungseinheit 16 in Verbindung steht,
- eine Strahlungseinrichtung 13 mit Licht im sichtbaren oder grünen Wellenlängenbereich zur Bestrahlung zumindest eines extraembryonalen Blutgefäßes 22, eines embryonalen Blutgefäßes 24 oder des Herzens 25 des Embryos 23,
- einen Detektor (z.B. Kamera) 14 für sichtbares oder grünes Licht 13a zur Erkennung zumindest eines extraembryonalen Blutgefäßes 22, eines embryonalen Blutgefäßes 24 oder des Herzens 25 des Embryos 23, wobei der Detektor 14 mit der Positionier- Auswerteeinrichtung 18 in Verbindung steht,
- eine Vorrichtung 6 zur Einbringung von Laserlicht 34 in das Ei 1,
die zumindest in Verbindung steht mit
- zwei oder mehreren Laserlicht 34 emittierenden Laserstrahlenquellen 35, 36,
- zwei oder mehreren Kollimatoren 41, 42 für jeweils jede Laserstrahlenquelle 35, 36,
- mindestens einem oder mehreren Strahleinkopplern 26 oder Spiegeln/semipermeablen Spiegeln 10 für die Laserstrahlenquelle 35, 36,
- zwei oder mehreren Detektoren 8 zur Aufnahme der Fluoreszenzstrahlung 5 in zwei oder mehreren spektralen Bereichen, die selektiert werden mittels Bandpassfilter 9,
- einer Steuereinheit 17 zu xyz-Positionierung der Vorrichtung 6 auf das in das Ei 1 eingebrachte Loch 2,
- eine Geschlechtsbestimmungs-Auswerteeinheit 19, die mit der Amplifikations- und Detektioneinheit 12 und der Positionier-Auswerteeinrichtung 18, die mit der Steuereinheit 17 verbunden ist, in Verbindung steht.

In der Konfiguration 3 gemäß Fig. 3 kann Folgendes durchgeführt werden:
- Die Anregung erfolgt mit zwei oder mehreren Wellenlängen. Die Anregungsstrahlung wird von zwei oder mehreren Lasern 35, 36 produziert.
- Der gesamte Spektralbereich oder ein spektraler Teilbereich der Fluoreszenz, der von jeder Anregungswellenlänge generiert wird, wird mit zwei oder mehreren Detekoren/Sensoren 8 und vorgegebenen Bandpassfiltern 9 registriert.
- Die registrierten und evtl. verstärkten Signalintensitäten I werden mathematisch ausgewertet, um das Geschlecht zu erhalten. Die mathematische Auswertung kann auf der Evaluation von Intensitäten, Intensitätsverhältnissen, - summen oder -differenzen basieren als auch überwachte Klassifikationsmethoden nutzen, wie die Diskriminanzanalyse, Support-Vektor-Maschinen oder neuronale Netzwerke.

Gemäß Fig. 7 können in der Auswerteeinheit 19 bei Einsatz mindestens zweier Laserstrahlenquellen 35, 36 die aus mehreren gemessenen Fluoreszenzintensitäts/Wellenzahl-Kurven 51, 52 ermittelten Intensitätsgrößen bezüglich eines vorgegebenen Spektralbereiches zwischen ca. 500 cm⁻¹ und ca. 3500 cm⁻¹ als die integralen Intensitäten I_{I511}, I_{I512} der Fluoreszenzstrahlungen 51 des männlichen Blutes und als die integralen Intensitäten I_{I521}, I_{I522} der Fluoreszenzstrahlungen 52 des weiblichen Blutes in Abhängigkeit von dem emittierten Wellenlängen der Laserstrahlenquellen 35, 36 festgelegt werden.

Damit soll die Sicherheit zur schnellen Bestimmung des Geschlechts aus den gleichzeitigen Fluoreszenzstrahlungsmessungen des jeweiligen Blutes erhöht werden.

Schließlich können in der Auswerteeinheit 19 bei Einsatz mindestes einer Laserstrahlenquelle 3 oder mehrerer Laserstrahlenquellen 35, 36 die aus mehreren ermittelten Fluoreszenzintensitäts/Wellenzahl-Kurven 51, 52 ermittelten Intensitätsgrößen bezüglich eines vorgegebenen Spektralbereiches jeweils für das männliche Blut und für das weibliche Blut kombiniert z.B. mittels logischer Verknüpfung in der Auswerteeinheit 19 zur Auswertung und zur schnellen Bestimmung des Geschlechts der Vogeleier 1 festgelegt werden.

Die Laserstrahlen 34, die in den Lasern 35, 36 produziert werden, können mittels Spiegeln oder mittels Faseroptik übertragen werden. Die Laserstrahlen 34 werden mit Kollimatoren 41, 42 gebündelt.

Die Laserstrahlen 34 werden überlappt mit einem Strahleinkoppler 26.

Die Laserstrahlen 34 werden zum Ei 1 übertragen und mit einer Linse/einem Objektiv 7 auf ein extraembryonales Blutgefäß 22, ein embryonales Blutgefäß 24 oder auf das Herz 25 des Embryos 23 fokussiert.

Die Fluoreszenzstrahlung 5 wird mit derselben den Laserfokus erzeugenden Linse/demselben den Laserfokus erzeugenden Objektiv 7 gesammelt.

Ein Strahlteiler 6 wird genutzt, um das Fluoreszenzsignal 5 vom sichtbaren Licht 13a der Strahlungsquelle 13 zu separieren.

Das Licht im sichtbaren Spektralbereich 13a wird zur Kamera 14 übertragen, ein Filter 15 entfernt verbleibende Strahlen des Laserlichtes.

Ein Strahlteiler 11 wird genutzt, um die Fluoreszenzstrahlung 5 zu separieren, welches auf die Detektoren 8 auftrifft. Die Detektoren 8, typischerweise jeweils eine Photodiode oder eine Lawinenphotodiode oder ein Photomultiplier, messen die Intensitäten der Fluoreszenzstrahlung 5.

Zwei oder mehrere vorgegebene Bandpassfilter 9 selektieren die Spektralbereiche der Fluoreszenzstrahlung, die mit jedem der Detektoren 8 registriert werden.

Eine Detektionseinheit 12 verstärkt, filtert und misst die Signale der Detektoren 8, welches zur Geschlechtsbestimmungs-Auswerteeinheit 19 gesendet wird. Um das Geschlecht zu bestimmen, können die Signalintensitäten I_{I511}, I_{I512}, I_{I521}, I_{I522} mit einer der folgenden Verfahren oder einer Kombination der folgenden Verfahren ausgewertet werden:
- Intensitäten werden mit Schwellwerten verglichen,
- Intensitätsverhältnisse werden mit Schwellwerten verglichen,
- Intensitätssummen oder -differenzen werden mit Schwellwerten verglichen,
- Überwachte Klassifikationsmethoden, wie beispielsweise Diskriminanzanalysen, Support-Vektor-Maschinen oder neuronale Netzwerke werden auf die Intensitätswerte angewendet.

Die Signalintensitäten können auch hier normiert werden, beispielsweise durch Vektor- oder Flächennormierung, und anschließend mathematisch mit einer der folgenden Methoden oder einer Kombination der folgenden Verfahren ausgewertet, um das Geschlecht zu erhalten:
- Normierte Intensitäten werden mit Schwellwerten verglichen,
- Verhältnisse zwischen normierten Intensitäten werden mit Schwellwerten verglichen,
- Summen oder Differenzen normierter Intensitäten werden mit Schwellwerten verglichen,
- Überwachte Klassifikationsmethoden wie beispielsweise Diskriminanzanalysen, Support-Vektor-Maschinen oder neuronale Netzwerke werden auf die normierten Intensitätswerte angewendet.

Als Klassifizierungsverfahren können die SVM - Supporting Vector Machine, LDA - Lineare Diskriminanz Analyse, KNN-Nearest-Neighbour Klassification oder ANN-Artificial Neural Networks Verfahren - eingesetzt werden. Auch andere Verfahren, wie z.B. nichtlineare Verfahren/Methoden oder unterstützende Einrichtungen oder SIMCA, können eingesetzt werden. Die Algorithmen klassifizieren die Intensitätswerte.
- wobei hierzu ein Referenzintensität Set mit Referenzintensitäten "männlich" und Referenzintensitäten "weiblich" mit bekannter geschlechtlicher Zuordnung benötigt wird. Der Algorithmus vergleicht das Referenzintensität Set mit anderen Referenzintensität Sets der Geschlechtsklasse und überprüft die Ähnlichkeit des unbekannten Referenzintensität-Sets mit den bekannten gespeicherten Referenzintensität-Sets.
- Schritt zur Ausgabe der Ergebnisse der Bestimmung des jeweiligen Geschlechts der Vogeleier, wobei das Ei 1 aussortiert wird, wenn die Mindestsicherheit für"männlich" gleich oder unter 45% erreicht wird. Andernfalls liegt ein weibliches Ei 1 vor, das weiter bebrütet wird.

In der zugehörigen Fig. 7 sind Fluoreszenzintensitäts/Wellenzahl-Kurven für die erfindungsgemäße dritte Vorrichtung nach Fig. 3 zur Bestimmung des Geschlechtes von bebrüteten Vogeleiern 1 auf der Basis entstehender Fluoreszenzstrahlung 5 nach Anregung mittels zwei unterschiedliche Wellenlängen emittierenden Laserstrahlenquellen 35, 36 mit Messung der unterschiedlichen integralen Intensitätskurven 51, 52 (jeweils durchgezogen, gestrichelt) gezeigt.

In der der Konfiguration 4 entsprechenden Fig. 4 ist in einer schematischen Darstellung eine vierte Vorrichtung 33 zur optischen in-ovo Geschlechtsbestimmung eines befruchteten und bebrüteten Vogeleies 1 auf der Basis der Rückstreustrahlung einschließlich der Fluoreszenzstrahlung 5 gezeigt, wobei die Vorrichtung 33 umfasst
- eine Ei-Lagerungseinheit 16, auf der das Ei 1 gelagert ist,
- eine Positions-Auswerteeinrichtung 17, die mit der Ei-Lagerungseinheit 16 in Verbindung steht,
- eine Strahlungseinrichtung 13 mit Licht im sichtbaren oder grünen Wellenlängenbereich zur Bestrahlung zumindest eines extraembryonalen Blutgefäßes 22, eines embryonalen Blutgefäßes 24 oder des Herzens 25 des Embryos 23,
- einen Detektor (z.B. Kamera) 14 für sichtbares oder grünes Licht 13a zur Erkennung zumindest eines extraembryonalen Blutgefäßes 22, eines embryonalen Blutgefäßes 24 oder des Herzens 25 des Embryos 23, wobei der Detektor 14 mit der Positionier-Auswerteeinrichtung 18 in Verbindung steht,
- eine Vorrichtung 6 zur Einbringung von Laserlicht 34 in das Ei 1,
die zumindest in Verbindung steht mit
- einer Laserlicht 34 emittierenden Laserquelle 3,
- einer Steuereinheit 17 zu xyz-Positionierung der Vorrichtung 6 auf das in das Ei 1 eingebrachte Loch 2,
- einem Spektrometer 20 zur Aufnahme der Fluoreszenzstrahlung 5 und der überlagernden Ramanstreustrahlung, wobei dem Spektrometer 20 ein Notchfilter/Kurzpassfilter 21 vorgeordnet ist, und
- eine Geschlechtsbestimmungs-Auswerteeinheit 19, die mit der Detektionseinheit 12 und der Positionier-Auswerteeinrichtung 18, die mit der Steuereinheit 17 verbunden ist, in Verbindung steht.

Im Wesentlichen unterscheiden sich die erfindungsgemäßen Vorrichtungen 30, 31, 32, 33 in den eingesetzten Bauelementen und Bauteilen zur Trennung der Rückstreustrahlung einschließlich der Fluoreszenzstrahlung 5 und auch der Ramanstreustrahlung 50, insbesondere auch in der Anzahl der Bandpassfilter 8, der Anzahl der Detektoren 8 und der Auswerteeinheit 19, je nachdem welche spektrale Intensität oder zugeordnete Größe vor allem der Fluoreszenzstrahlung 5 in der Auswerteeinheit 19 ausgewertet wird, und welche programmtechnischen Mittel in der Auswerteeinheit 19 zur vorgesehenen Geschlechtsbestimmung enthalten sind.

In der Konfiguration 4 gemäß Fig. 4 mit der Registrierung der Fluoreszenzstrahlung 5, 51, 52 und der Ramanstreustrahlung 50 erfolgt Folgendes:
- Die Anregung erfolgt bei einer Wellenlänge (Laser 3).
- Der gesamte Spektralbereich oder ein spektraler Teilbereich der Fluoreszenz und Rückstreuung wird registriert und einem Spektrometer 20 zugeführt.
- Aus dem registrierten Spektrum, welches aus einer Überlagerung von Fluoreszenz und inelastischer Ramanrückstreuung besteht, wird die Geschlechtsinformation extrahiert. Das registrierte Spektrum im Spektralbereich von etwa 100 cm⁻¹ - 4000 cm⁻¹ oder ein spektraler Teilbereich daraus wird direkt ausgewertet oder es wird eine Normierung der Daten durchgeführt. Anschließend werden überwachte Klassifikationsmethoden, wie die Diskriminanzanalyse, Support-Vektor-Maschinen oder neuronale Netzwerke angewendet, um die Geschlechtsinformation zu erhalten.

Unter Berücksichtigung der ebenfalls auftretenden und registrierten Ramanstreustrahlung können gemäß Fig. 8 in der Auswerteeinheit 19 die aus den gemessenen Fluoreszenzintensitäts/Wellenzahl-Kurven 51, 52 ermittelten Intensitätsgrößen bezüglich eines vorgegebenen Spektralbereiches als die integrale Intensität I_{I51} der Fluoreszenzstrahlung 51 des männlichen Blutes und als die integrale Intensität I_{I52} der Fluoreszenzstrahlung 52 des weiblichen Blutes und die jeweils die Fluoreszenzstrahlungen 51, 52 überlagernden Ramanstreustrahlungen 50₅₁, 50₅₂ für die Auswertung zur schnellen Bestimmung des Geschlechts des Vogelblutes festgelegt werden.

Das in Fig. 8 eingezeichnete Rechteck 53 stellt den ausgewählten Spektralbereich dar, der im angegebenen Beispiel für die Klassifizierung verwendet wird.

In der zugehörigen Fig. 8 sind dazu Fluoreszenzintensitäts/Wellenzahl-Kurven für die erfindungsgemäße vierte Vorrichtung 33 zur Bestimmung des Geschlechtes von bebrüteten Vogeleiern 1 auf der Basis entstehender Fluoreszenzstrahlung 5 und inelastischer Ramanstreustrahlung 50 nach Anregung mit einer Laserstrahlenquelle 3 zur Messung der beiden sich überlagernden Intensitäten 51 und 50, 52 und 50 gezeigt.

Die auf die vierte Vorrichtung 33 bezogene, in Fig. 8 dargestellte, registrierte spektrale inelastische Rückstreustrahlung ist variabel im Spektralbereich, aber immer rotverschoben im Vergleich zur Anregungswellenlänge des Lasers 3. Typisch ist eine Wellenzahlverschiebung zwischen ca. 500 cm⁻¹ and 3500 cm⁻¹. In dem angegebenen Spektralbereich 53 (Rechteck) ist die Autofluoreszenz des Blutes für männliche Embryonen und für weibliche Embryonen unterschiedlich. Die Autofluoreszenz des männlichen Blutes weist einen höheren Intensitätsverlauf 51 als der Intensitätsverlauf 52 bezüglich des weiblichen Blutes auf.

Die deutlich schwächere Ramanstreustrahlung 50₅₁, 50₅₂ der männlichen Embryonen und weiblichen Embryonen ist jeweils den Fluoreszenzstrahlungen 51, 52 überlagert.

Die spektroskopische Auswertung erfolgt in der Auswerteeinheit 19 unter Einbeziehung mathematischer Klassifizierungsalgorithmen.

Für die Durchführung der spektralen Klassifizierung und deren Ergebnisausgabe wird ein mehrstufiger Prozess angegeben:
1. Schritt der Messung der Spektren des zu untersuchenden Eies 1,
2. Schritt der Datenvorbehandlung mit
   - einer Reduzierung des spektralen Bereiches der Rückstrahlung auf einen Spektralbereich zwischen 500 cm⁻¹ und 4000 cm⁻¹ oder kleiner.
   - einer Normierung der Spektren auf eine integrale Intensität durch Flächen- oder Vektornormierung, die evtl. durchgeführt werden kann, aber nicht zwingend notwendig ist.
3. Schritt der spektralen Klassifizierung mit
   der Anwendung einer unterstützten (engl. supervised) Klassifizierung. Als Klassifizierungsverfahren können die SVM - Supporting Vector Machine, LDA - Lineare Diskriminanz Analyse, KNN-Nearest-Neighbour Klassification oder ANN-Artificial Neural Networks Verfahren - eingesetzt werden. Auch andere Verfahren, wie z.B. nichtlineare Verfahren/Methoden oder unterstützende Einrichtungen oder SIMCA, können eingesetzt werden. Die LDA klassifiziert mehrere spektrale Bereiche, also die Intensitätswerte dieser Bereiche,
   gegebenenfalls mit einem Schritt der Verifizierung, wobei hierzu ein Referenzspektren-Set mit Referenzspektren "männlich" und Referenzspektren "weiblich" mit bekannter geschlechtlicher Zuordnung benötigt wird. Der Algorithmus vergleicht das Spektrum mit anderen Spektren der Geschlechtsklasse und überprüft die Ähnlichkeit des unbekannten Spektrums mit den bekannten gespeicherten Referenzspektren.

Nach dem Schritt zur Ausgabe und der Anzeige der Ergebnisse zur Bestimmung des jeweiligen Geschlechts der Vogeleier wird dann das Ei 1 aussortiert, wenn die Mindestsicherheit für "männlich" gleich oder unter 45% erreicht wird. Andernfalls liegt ein weibliches Ei 1 vor, das weiter bebrütet wird.

In Fig. 8a sind die normierten Intensitäten beider Fluoreszenzstrahlungen 51, 52 dargestellt. Durch die Normierung wird sichtbar, dass die Spektren des männlichen Blutes im Vergleich zu den Spektren des weiblichen Blutes ein unterschiedliches Spektralprofil aufweisen.

Im Folgenden werden durchgeführte Ausführungsbeispiele und die Ergebnisse im Detail erläutert:
Eine 15 mm Öffnung wurde am spitzen Pol von 165 Hühnereiern am Tag 3,5 der Inkubation eingebracht. Das Referenz-Geschlecht der Eier 1 wurde durch nachfolgende PCR-Bestimmung erhalten. Die PCR-Bestimmung ergab, dass 80 Eier einen männlichen Embryo und 85 Eier einen weiblichen Embryo enthielten. Ein extraembryonales Blutgefäß 22 wurde unter grüner LED-Beleuchtung (500 nm - 550 nm) und mittels einer Kamera 14 unter hoher Vergrößerung selektiert.

Ein CW-Laserstrahl der Laserstrahlenquelle 3 (Leistung: 200 mW; Anregungswellenlänge: 785 nm) wurde auf ein extraembryonales Blutgefäß 22 mittels des Objektives 7 (numerische Apertur NA=0.4) fokussiert.

Die vorgenannten Vorrichtungen 30, 31, 32, 33 arbeiten zumindest nach einem der folgend genannten Verfahren:
In den erfindungsgemäßen Verfahren zur optischen in-ovo Geschlechtsbestimmung von befruchteten und bebrüteten Vogeleiern 1 werden folgende Schritte durchgeführt:
- Überwachung des Zeitverlaufs des Bebrütens bis zur Ausbildung mindestens eines erkennbaren Blutgefäßes 22, 24 oder des Herzens 25 mit fließendem Blut,
- Schaffung eines Lochs 2 in der Kalkschale 28 des Vogeleies 1 mittels einer Locherzeugungseinheit,
- Suchen der sich im Ei 1 ausbildenden Blutgefäße 22, 24 oder des Herzens 25 mittels eines Visions-Systems 13, 13a und einer koaxialen oder lateralen Beleuchtung mit Licht im sichtbarem Wellenlängenbereich,
- Positionieren zumindest eines Blutgefäßes 22, 24 oder des Herzens 25 in den Laserfokus mindestens einer Laserstrahlenquelle 3; 35, 36 entweder durch Bewegen des Eies 1 oder durch Bewegen des den Laserfokus erzeugenden Objektives 7,
- Bestrahlung des Blutgefäßes 22, 24 oder des Herzens 25 mit mindestens einer eine Anregungswellenlänge emittierenden Laserstrahlenquelle 3, 35, 36,
- Registrieren der Rückstreustrahlung 5, 50, 51, 52 des bestrahlten Blutgefäßes 22, 24 oder des Herzens 25 mittels mindestens eines Detektors 8, der mit mindestens einer mit einer nachgeschalteten Amplifikations- und Detektoreinheit 12 verbundenen Auswerteeinheit 19 in Verbindung steht, wobei während der Registrierung bei einer Bewegung des ausgewählten Blutgefäßes 22, 24 oder des Herzens 25 aus dem Laserstrahl 3b heraus eine Nachführung der Blutgefäße 22, 24 oder des Herzens 25 oder des den Laserfokus erzeugenden Objektivs/der Linse 7 erfolgen kann.

Erfindungsgemäß werden nachfolgend folgende Schritte durchgeführt:
- Auswertung der Rückstreustrahlung 5, 50, 51, 52 einschließlich der Fluoreszenzstrahlung 5, 51, 52 in der Auswerteeinheit 19 aus der registrierten spektralen Intensität der Fluoreszenzstrahlung 51, 52 in einem zur Anregungswellenlänge rotverschobenen Spektralbereich, wobei die geschlechtsspezifischen Eigenschaften des männlichen Blutes und des weiblichen Blutes in der Intensität und im Spektralprofil der registrierten Fluoreszenzstrahlung 51, 52 enthalten sind und wobei zumindest eine der aus den gemessenen spektralen Intensitäten der Fluoreszenzstrahlung 51, 52 ermittelten Intensitätsgrößen oder deren zugeordnete Größen bezüglich des männlichen Blutes gegenüber zumindest einer der ermittelten Intensitätsgrößen oder deren zugeordnete Größen bezüglich des weiblichen Blutes in den Blutgefäßen 22, 24 oder im Herz 25 einen auswertbaren unterschiedlichen Wert aufweisen,
- Bestimmung des Geschlechts des Vogeleies 1 aus dem Unterschied zumindest eines der Werte der Fluoreszenzintensitätsgrößen 51, 52 oder deren zugeordnete Größen in der Auswerteeinheit 19 und danach zumindest
- eine Anzeige des in der Auswerteeinheit 19 bestimmten Geschlechts des Embryos 23 im Vogelei 1.

Das Licht im sichtbaren Wellenlängenbereich, mit dem die Strahlungseinrichtung 13 des Visions-Systems zur Bestrahlung zumindest eines Blutgefäßes 22, 24 oder des Herzens 25 vorgesehen ist, kann vorzugsweise grünes Licht darstellen.

Der jeweils auswertbare unterschiedliche Wert der Intensitätsgrößen kann sich zumindest auf einen vorgegebenen, in der Auswerteinheit 19 gespeicherten, der Intensitätsgröße zugeordneten Grenz-/Schwellwert beziehen.

In der Auswerteeinheit 19 können die aus den ermittelten Fluoreszenzintensitäts/Wellenzahl-Kurven 51, 52 ermittelten Intensitätsgrößen bezüglich eines vorgegebenen Spektralbereiches als die integrale Intensität I_{I51} der Fluoreszenzstrahlung 51 des männlichen Blutes und als die integrale Intensität I_{I52} der Fluoreszenzstrahlung 52 des weiblichen Blutes festgelegt werden.

Auch können in der Auswerteeinheit 19 die aus den ermittelten Fluoreszenzintensitäts/Wellenzahl-Kurven 51, 52 ermittelten Intensitätsgrößen bezüglich eines vorgegebenen Spektralbereiches als das Intensitätsmaximum 51_{Max} der Fluoreszenzstrahlung 51 des männlichen Blutes und als das Intensitätsmaximum 52_{Max} der Fluoreszenzstrahlung 52 des weiblichen Blutes festgelegt werden.

Des Weiteren können in der Auswerteinheit 19 die aus den ermittelten Fluoreszenzintensitäts/Wellenzahl-Kurven 51, 52 ermittelten Intensitätsgrößen I_{D1} bis I_{D8} bezüglich vorgegebener und nebeneinander geordneter Spektralbereiche als integrale Intensität der Fluoreszenzstrahlung 51 des männlichen Blutes und als integrale Intensität der Fluoreszenzstrahlung 52 des weiblichen Blutes innerhalb der vorgegebenen Spektralbereiche, bei der z.B. die Differenzen der jeweiligen Intensitätsgrößen I_{D151} bis I_{D852} am größten ist, festgelegt werden.

In der Auswerteeinheit 19 können bei Einsatz einer Laserstrahlenquelle 3 die aus der ermittelten Fluoreszenzintensitäts/Wellenzahl-Kurve 51, 52 ermittelten Intensitätsgrößen bezüglich eines vorgegebenen Spektralbereiches jeweils für das männliche Blut und für das weibliche Blut mittels logischer Verknüpfung kombiniert zur Auswertung festgelegt werden.

Dabei können in der Auswerteeinheit 19 bei Einsatz mindestes zweier Laserstrahlenquellen 35, 36 die aus mehreren ermittelten Fluoreszenzintensitäts/Wellenzahl-Kurven 51, 52 ermittelten Intensitätsgrößen bezüglich eines vorgegebenen Spektralbereiches als die integralen Intensitäten I_{I511}, I_{I512} der Fluoreszenzstrahlungen 51 des männlichen Blutes und als die integralen Intensitäten I_{I521}, I_{I522} der Fluoreszenzstrahlungen 52 des weiblichen Blutes festgelegt werden.

In der Auswerteeinheit 19 können bei Einsatz mindestes zweier Laserstrahlenquellen 35, 36 die aus mehreren ermittelten Fluoreszenzintensitäts/Wellenzahl-Kurven 51, 52 ermittelten Intensitätsgrößen bezüglich eines vorgegebenen Spektralbereiches jeweils für das männliche Blut und für das weibliche Blut kombiniert zur Auswertung festgelegt werden.

In der Auswerteeinheit 19 können die aus den ermittelten Fluoreszenzintensitäts/Wellenzahl-Kurven 51, 52 ermittelten Intensitätsgrößen bezüglich eines vorgegebenen Spektralbereiches als die integrale Intensität I_{I51} der Fluoreszenzstrahlung 51 des männlichen Blutes und als die integrale Intensität I_{I52} der Fluoreszenzstrahlung 52 des weiblichen Blutes und die jeweils die Fluoreszenzstrahlungen 51, 52 überlagernden Ramanstreustrahlungen 50₅₁, 50₅₂ für die Auswertung allein oder in Kombination mit den anderen festgelegten Intensitätsgrößen und zugeordneten Größen festgelegt werden, wobei bei Kombination der Intensitätsgrößen und zugeordneter Größen die Ramanstreustrahlung und die Fluoreszenzstrahlung mittels logischer Verknüpfung bzw. hardwaremäßig durch Verknüpfungsglieder gemeinsam ausgewertet werden.

Die Auswerteeinheit (19) kann zur logischen Verknüpfung von Intensitätsgrößen und zugeordneten Größen oder aus den Intensitätsgrößen abgeleiteten Größen programmtechnische Mittel zur Durchführung von mindestens einer logischen Verknüpfung und/oder als Hardware ausgebildete logische Verknüpfungsglieder (AND und andere) zur Durchführung von mindestens einer logischen Verknüpfung aufweisen.

Bei Einsatz einer gepulsten Laserstrahlung aus der Laserstrahlenquelle 3 kann die erzeugte Fluoreszenzintensität zeitaufgelöst gemessen und aus der Zeitkonstanten *τ* der Abklingkurve der zeitaufgelösten Fluoreszenzintensität eine Geschlechtsbestimmung durchgeführt werden, wobei für männliches Blut und für weibliches Blut jeweils eine unterschiedliche Zeitkonstante *τ*_{männlich}, *τ*_{weibllich} mit *τ*_{männlich} ≠ *τ*_{weibllich} zur Geschlechtsbestimmung ermittelt wird.

Im Folgenden werden schließlich Beispiele (Bsp.) für die Erkennungssicherheit des Geschlechts angegeben:

### Auswertung Bsp. 1:

Die Fluoreszenzintensität 51, 52 (5) wurde im Bereich zwischen 807 nm - 1000 nm registriert (entspricht einer Frequenzverschiebung im Bereich zwischen 350 cm⁻¹ - 2750 cm⁻¹), wie in Fig. 5 gezeigt ist.

Der angepasste Schwellwert SW für die Intensität, bei der die Geschlechter separiert werden können, wurde mit 1.06 * 10^7 counts/s (Zählersignale/Sekunde) am Detektor 8 gefunden. 70% der männlichen Enbryonen hatten eine Intensität über dem angepassten Schwellwert SW und 81% der weiblichen Embryonen unter dem angepassten Schwellwert SW, wie in Fig. 5a gezeigt ist.

### Auswertung Bsp. 2:

Der registrierte Fluoreszenzbereich wurde auf einen Bereich von 970 nm - 1000 nm begrenzt (entspricht einer Frequenzverschiebung im Bereich 2430 cm⁻¹ - 2750 cm⁻¹), gemäß der Fig. 5b und Fig. 5c.

Der Schwellwert SW für die Intensität, bei der die Geschlechter separiert werden können, wurde mit 8.25 * 10^5 counts/s (Zählersignale/Sekunde) am Detektor 8 gefunden. 73% der männlichen Embryonen hatten eine Intensität über diesem Schwellwert SW und 84% der weiblichen Embryonen unter diesem Schwellwert SW, gemäß der Fig. 5c.

### Auswertung Bsp. 3:

Die Fluoreszenzstrahlung 5 wurde, wie in Fig. 6 gezeigt ist, in acht separaten Spektralbereichen D1, D2, D3, D4, D5, D6, D7, D8 jeweils mit einer Breite von 300 cm⁻¹ im Bereich zwischen 350 cm⁻¹ bis 2750 cm⁻¹ registriert. Die registrierten Intensitäten gemäß Fig. 6a wurden einer linearen Diskriminanzanalyse zugeführt. 75% der männlichen Embryos und 95% der weiblichen Embryos wurden korrekt klassifiziert.

### Auswertung Bsp. 4:

Die Fluoreszenzstrahlung 5, 51, 52 wurde, wie in Fig. 6 und Fig. 6a gezeigt ist, in acht separaten Spektralbereichen D1, D2, D3, D4, D5, D6, D7, D8 jeweils mit einer Breite von 300 cm⁻¹ im Frequenzbereich zwischen 350 cm⁻¹ to 2750 cm⁻¹ registriert. Die registrierten Intensitäten wurden vektornormiert und einer Diskriminanzanalyse zugeführt, wie in Fig. 6b gezeigt ist. 86% der männlichen Embryos und 91% der weiblichen Embryos wurden korrekt klassifiziert.

### Auswertung Bsp. 5

Die Fluoreszenzstrahlung 5, 51, 52 und die Ramanstreustrahlung 50 wurden registriert und zu einem Spektrometer 20 gesendet. Die Spektren in dem Spektralbereich 600 cm⁻¹ - 1500 cm⁻¹ (Rechteck 53) wurden einer linearen Diskriminanzanalyse gemäß Fig. 8 zugeführt. 94% der männlichen Embryos und 88% der weiblichen Embryos wurden korrekt klassifiziert.

### Auswertung Bsp. 6:

Die Fluoreszenzstrahlung 5, 51, 52 gemäß Fig.8 und die Ramanstreustrahlung 50 wurden registriert und zu einem Spektrometer 20 gesendet. Die Spektren in dem Spektralbereich 600 cm⁻¹ - 1500 cm⁻¹ wurden gemäß Fig. 8a normiert und einer linearen Diskriminanzanalyse zugeführt. 85% der männlichen Embryos und 80% der weiblichen Embryos wurden korrekt klassifiziert.

Die Vorteile der erfindungsgemäßen Geschlechtsbestimmung sind:
- keine Beeinträchtigung des Schlupfes und der nachfolgenden Entwicklung des Kükens und
- Durchführung der Geschlechtsbestimmung mit hoher Genauigkeit in Echtzeit zu einem sehr frühen Zeitpunkt und mithilfe einer kontaktlosen Bestimmung ohne Probenentnahme.

### Bezugszeichenliste

1 Ei
2 Loch in der Ei-Kalkschale 28
3 Laserstrahlenquelle
3b in das Loch 2 der Kalkschale 28 eingebrachtes Laserlicht
4 Kollimator
5 Strahlung zur Detektion/Fluoreszenzstrahlung
6 Vorrichtung zur Einbringung der Laserstrahlung in das Ei (Strahlteiler)
7 Linse/Objektiv zur Fokussierung der Laserstrahlung und zum Sammeln der Fluoreszenzstrahlung
8 Detektor/en
9 Detektions-Filter/Bandpassfilter
10 Detektions-Strahlteiler/Spiegel
11 Vorrichtung zur Separation der Fluoreszenz (Strahlteiler)
12 Amplifikations- und Detektionseinheit
13 Lichtquelle/Strahlungseinrichtung des Visions-Systems VIS 13a Transmittertes bzw. gestreutes sichtbares Licht
14 Detektor/Kamera zur Detektion von Licht 13a
15 Kamerafilter
16 xyz-Positioniereinheit zur Ei-Lagesteuerung
16a xyz-Positioniereinheit zur Linsen-Lagesteuerung
17 Steuereinheit
18 Positionier-Auswerteinheit
19 Auswerteeinheit/Geschlechtsbestimmungs-Auswerteeinheit
20 Spektrometer
21 Notchfilter/Kurzpassfilter
22 extraembryonales Blutgefäß
23 Embryo
24 embryonales Blutgefäß
25 Herz
26 Strahleinkoppler
28 Kalkschale
30 erste Vorrichtung
31 zweite Vorrichtung
32 dritte Vorrichtung
33 vierte Vorrichtung
34 Laserlicht
35 erste Laserstrahlenquelle
36 zweite Laserstrahlenquelle
41 erster Kollimator
42 zweiter Kollimator
50 Ramanstreustrahlung
50₅₁ Ramanstreustrahlung des männlichen Blutes
50₅₂ Ramanstreustrahlung des weiblichen Blutes
51 Fluoreszenzintensitätsverlauf des männlichen Blutes
52 Fluoreszenzintensitätsverlauf des weiblichen Blutes
53 Rechteck
I_{I51}, I_{I51} Integrale Intensitäten
I_{I511}, I_{I512}, I_{I521}, I_{I522} Integrale Intensitäten
51_{Max}, 52_{Max} Intensitätsmaxima der Fluoreszenzintensitätskurven
D1 ... D8 vorgegebene, anteilige Spektralbereiche
T Zeitkonstante
♂ männlich
♀ weiblich

## Patentansprüche

1. Verfahren zur optischen in-ovo Geschlechtsbestimmung von befruchteten und bebrüteten Vogeleiern (1) mit folgenden Schritten:
- Überwachung des Zeitverlaufs des Bebrütens bis zur Ausbildung mindestens eines erkennbaren Blutgefäßes (22, 24) oder des Herzens (25) mit fließendem Blut,
- Schaffung eines Lochs (2) in der Kalkschale (28) des Vogeleies (1) mittels einer Locherzeugungseinheit,
- Suchen der sich im Ei (1) ausbildenden Blutgefäße (22, 24) oder des Herzens (25) mittels eines Visions-Systems (13, 13a) und einer koaxialen oder lateralen Beleuchtung mit Licht im sichtbaren Wellenlängenbereich,
- Positionieren zumindest eines Blutgefäßes (22, 24) oder des Herzens (25) in den Laserfokus mindestens einer Laserstrahlenquelle (3; 35, 36) entweder durch Bewegen des Eies (1) oder durch Bewegen eines den Laserfokus erzeugenden Objektivs (7),
- Bestrahlung des Blutgefäßes (22, 24) oder des Herzens (25) mit mindestens einer eine Anregungswellenlänge emittierenden Laserstrahlenquelle (3, 35, 36),
- Registrieren der Rückstreustrahlung (5, 50, 51, 52) des bestrahlten Blutgefäßes (22, 24) oder des Herzens (25) mittels mindestens eines Detektors (8), der mit mindestens einer mit einer nachgeschalteten Amplifikations- und Detektoreinheit (12) verbundenen Auswerteeinheit (19) in Verbindung steht, wobei während der Registrierung eine Bewegung des Blutgefäßes (22, 24) oder des Herzens (25) aus dem Laserstrahl (3b) heraus durch Nachführung der Blutgefäße (22, 24) oder des Herzens (25) oder des Objektivs (7) erfolgen kann,
**gekennzeichnet durch** folgende Schritte:
- Auswertung der Rückstreustrahlung (5, 50, 51, 52) einschließlich der Fluoreszenzstrahlung (5, 51, 52) in der Auswerteeinheit (19) aus der registrierten spektralen Intensität der Fluoreszenzstrahlung (51, 52) in einem zur Anregungswellenlänge rotverschobenen Spektralbereich, wobei die geschlechtsspezifischen Eigenschaften des männlichen Blutes und des weiblichen Blutes in der Intensität und im Spektralprofil der registrierten Fluoreszenzstrahlung (51, 52) enthalten sind und wobei zumindest eine der aus den gemessenen spektralen Intensitäten der Fluoreszenzstrahlung (51, 52) ermittelten Intensitätsgrößen oder deren zugeordnete Größen bezüglich des männlichen Blutes gegenüber zumindest einer der ermittelten Intensitätsgrößen oder deren zugeordnete Größen bezüglich des weiblichen Blutes in den Blutgefäßen (22, 24) oder im Herz (25) einen auswertbaren unterschiedlichen Wert aufweisen,
- Bestimmung des Geschlechts des Vogeleies (1) aus dem Unterschied zumindest eines der Werte der Fluoreszenzintensitätsgrößen (51, 52) oder deren zugeordnete Größen in der Auswerteeinheit (19) und danach zumindest
- eine Anzeige des in der Auswerteeinheit (19) bestimmten Geschlechts des Embryos (23) im Vogelei (1).

2. Verfahren nach Anspruch 1,
**dadurch gekennzeichnet,**
**dass** als Licht im sichtbaren Wellenlängenbereich, mit dem eine Strahlungseinrichtung (13) des Visions-Systems zur Bestrahlung zumindest eines Blutgefäßes (22, 24) oder des Herzens (25) vorgesehen wird, grünes Licht verwendet wird.

3. Verfahren nach Anspruch 1,
**dadurch gekennzeichnet,**
**dass** die Fluoreszenzintensitäten I_{I511}, I_{I512}, I_{I521}, I_{I522} mit einer der folgenden Verfahren oder einer Kombination der folgenden Verfahren in der Auswerteeinheit (19) ausgewertet werden, um das Geschlecht zu erhalten:
- Intensitäten werden mit Schwellwerten (SW) verglichen,
- Intensitätsverhältnisse werden mit Schwellwerten (SW) verglichen,
- Intensitätssummen oder -differenzen werden mit Schwellwerten (SW) verglichen,
- Überwachte Klassifikationsmethoden, wie beispielsweise Diskriminanzanalysen, Support-Vektor-Maschinen oder neuronale Netzwerke werden auf die Intensitätswerte angewendet.

4. Verfahren nach Anspruch 1,
**dadurch gekennzeichnet,**
**dass** Fluoreszenzintensitäten normiert werden, beispielsweise durch Vektor- oder Flächennormierung, und anschließend mathematisch mit einer der folgenden Methoden oder einer Kombination der folgenden Verfahren in der Auswerteeinheit (19) ausgewertet werden, um das Geschlecht zu erhalten:
- Normierte Intensitäten werden mit Schwellwerten verglichen,
- Verhältnisse zwischen normierten Intensitäten werden mit Schwellwerten verglichen,
- Summen oder Differenzen normierter Intensitäten werden mit Schwellwerten verglichen,
- Überwachte Klassifikationsmethoden, wie beispielsweise Diskriminanzanalysen, Support-Vektor-Maschinen oder neuronale Netzwerke werden auf die normierten Intensitätswerte angewendet.

5. Verfahren nach Anspruch 1,
**dadurch gekennzeichnet,**
**dass** sich der jeweils auswertbare unterschiedliche Wert der Intensitätsgrößen zumindest auf einen gemessenen Schwellwert (SW) oder einen vorgegebenen, in der Auswerteinheit (19) gespeicherten, der Intensitätsgröße zugeordneten Schwellwert (SW) bezieht.

6. Verfahren nach Anspruch 1,
**dadurch gekennzeichnet,**
**dass** in der Auswerteeinheit (19) die aus den ermittelten Fluoreszenzintensitäts/Wellenzahl-Kurven (51, 52) ermittelten Intensitätsgrößen bezüglich eines vorgegebenen Spektralbereiches als die integrale Intensität I_{I51} der Fluoreszenzstrahlung (51) des männlichen Blutes und als die integrale Intensität I_{I52} der Fluoreszenzstrahlung (52) des weiblichen Blutes festgelegt werden.

7. Verfahren nach Anspruch 1,
**dadurch gekennzeichnet,**
**dass** in der Auswerteeinheit (19) die aus den ermittelten Fluoreszenzintensitäts/Wellenzahl-Kurven (51, 52) ermittelten Intensitätsgrößen bezüglich eines vorgegebenen Spektralbereiches als das Intensitätsmaximum 51_{Max} der Fluoreszenzstrahlung (51) des männlichen Blutes und als das Intensitätsmaximum 52_{Max} der Fluoreszenzstrahlung (52) des weiblichen Blutes festgelegt werden.

8. Verfahren nach Anspruch 1,
**dadurch gekennzeichnet,**
**dass** in der Auswerteinheit (19) die aus den ermittelten Fluoreszenzintensitäts/Wellenzahl-Kurven (51, 52) ermittelten Intensitätsgrößen I_{D1} bis I_{D8} bezüglich mehrerer vorgegebener, nebeneinander angeordneter Spektralbereiches (D1 bis D8) als integrale Intensität der Fluoreszenzstrahlung (51) des männlichen Blutes und als integrale Intensität der Fluoreszenzstrahlung (52) des weiblichen Blutes innerhalb der vorgegebenen Spektralbereiche (D1 bis D8) festgelegt werden.

9. Verfahren nach den Ansprüchen 2 bis 8,
**dadurch gekennzeichnet,**
**dass** in der Auswerteeinheit (19) bei Einsatz einer Laserstrahlenquelle (3) die aus der ermittelten Fluoreszenzintensitäts/Wellenzahl-Kurve (51, 52) ermittelten Intensitätsgrößen bezüglich eines vorgegebenen Spektralbereiches jeweils für das männliche Blut und für das weibliche Blut mittels logischer Verknüpfung kombiniert mittels logischer Verknüpfung zur Auswertung festgelegt werden.

10. Verfahren nach den Ansprüchen 1 bis 4,
**dadurch gekennzeichnet,**
**dass** in der Auswerteeinheit (19) bei Einsatz mindestes zweier Laserstrahlenquellen (35, 36) die aus mehreren ermittelten Fluoreszenzintensi- täts/Wellenzahl-Kurven (51, 52) ermittelten Intensitätsgrößen bezüglich eines vorgegebenen Spektralbereiches als die integralen Intensitäten I_{I511}, I_{I512} der Fluoreszenzstrahlungen (51) des männlichen Blutes und als die integralen Intensitäten I_{I521}, I_{I522} der Fluoreszenzstrahlungen (52) des weiblichen Blutes festgelegt werden.

11. Verfahren nach den Ansprüchen 2 bis 10,
**dadurch gekennzeichnet,**
**dass** in der Auswerteeinheit (19) bei Einsatz mindestes zweier Laserstrahlenquellen (35, 36) die aus mehreren ermittelten Fluoreszenzintensitäts/Wellenzahl-Kurven (51, 52) ermittelten Intensitätsgrößen bezüglich eines vorgegebenen Spektralbereiches jeweils für das männliche Blut und für das weibliche Blut kombiniert mittels logischer Verknüpfung zur Auswertung festgelegt werden.

12. Verfahren nach Anspruch 1,
**dadurch gekennzeichnet,**
**dass** in der Auswerteeinheit (19) die aus den ermittelten Fluoreszenzintensitäts/Wellenzahl-Kurven (51, 52) ermittelten Intensitätsgrößen bezüglich eines vorgegebenen Spektralbereiches als die integrale Intensität I_{I51} der Fluoreszenzstrahlung (51) des männlichen Blutes und als die integrale Intensität I_{I52} der Fluoreszenzstrahlung (52) des weiblichen Blutes und die jeweils die Fluoreszenzstrahlungen (51, 52) überlagernden Ramanstreustrahlungen (50₅₁, 50₅₂) für die Auswertung allein oder in Kombination mit den anderen festgelegten Intensitätsgrößen und zugeordneten Größen festgelegt werden, wobei bei Kombination der Intensitätsgrößen und zugeordneter Größen die Ramanstreustrahlung und die Fluoreszenzstrahlung mittels logischer Verknüpfung gemeinsam ausgewertet werden.

13. Verfahren nach Anspruch 1,
**dadurch gekennzeichnet,**
**dass** bei Einsatz einer gepulsten Laserstrahlung aus der Laserstrahlenquelle (3) die erzeugte Fluoreszenzintensität zeitaufgelöst gemessen und aus der Zeitkonstanten *τ* der Abklingkurve der zeitaufgelösten Fluoreszenzintensität eine Geschlechtsbestimmung durchgeführt wird, wobei für männliches Blut und für weibliches Blut jeweils eine unterschiedliche Zeitkonstante *τ*_{männlich}, *τ*_{weibllich} mit *τ*_{männlich} ≠ *τ*_{weibllich} zur Geschlechtsbestimmung ermittelt wird.

14. Vorrichtung (30, 31, 32, 33) zur optischen in-ovo Geschlechtsbestimmung von befruchteten und bebrüteten Vogeleiern (1) auf der Basis entstehender Rückstreustrahlung unter Einsatz des Verfahrens nach den Ansprüchen 1 bis 13, wobei die Vorrichtung (30) umfasst
- eine Ei-Lagerungseinheit (16), auf der das Ei (1) gelagert ist,
- eine Positions-Auswerteeinrichtung (17), die mit der Ei-Lagerungseinheit (16) in Verbindung steht,
- eine Strahlungseinrichtung (13) mit Licht im sichtbaren Wellenlängenbereich zur Bestrahlung zumindest eines Blutgefäßes (22, 24) oder des Herzens (25) des Embryos (23),
- einen Detektor (14) für sichtbares oder grünes Licht (13a) zur Erkennung zumindest eines Blutgefäßes (22, 24) oder des Herzens (25) des Embryos (23), wobei der Detektor (14) mit der Positionier-Auswerteeinrichtung (18) in Verbindung steht,
- eine Vorrichtung (6) zur Einbringung von Laserlicht (34) in das Ei (1),
die zumindest umfasst
a) mindestens eine Laserlicht (34) emittierenden Laserquelle (3),
b) mindestens einen Detektor (8) zur Aufnahme der Fluoreszenzstrahlung (5),
c) eine Steuereinheit (17) zu xyz-Positionierung der Vorrichtung (6) auf das in das Ei (1) eingebrachte Loch (2),
d) eine Geschlechtsbestimmungs-Auswerteeinheit (19), die mit der Amplifikations- und Detektionseinheit (12) und der Positionier-Auswerteeinrichtung (18), die mit der Steuereinheit (17) verbunden ist, in Verbindung steht,
**dadurch gekennzeichnet,**
**dass** eine Vorrichtung (11) zur Separation der Fluoreszenzstrahlung (5) aus der Rückstreustrahlung zwischen einem Kollimator (4) für die Strahlbündelung des Laserstrahls (34) und der Vorrichtung zur Einbringung des Laserlichts (34) in das Ei (1) angeordnet ist, wobei zwischen der strahlseparierenden Vorrichtung (11) und mindestens einem Fluoreszenz-Detektor (8) jeweils ein strahlengangzugehöriger Detektionsfilter (9) für die Transmission der Fluoreszenzstrahlung (5) eingebracht ist,
wobei in der Geschlechtsbestimmungs-Auswerteeinheit (19) erfolgen
- eine Auswertung der Rückstreustrahlung (5, 50, 51, 52) einschließlich der Fluoreszenzstrahlung (5, 51, 52) aus der registrierten spektralen Intensität der Fluoreszenzstrahlung (51, 52) in einem zur Anregungswellenlänge rotverschobenen Spektralbereich, wobei die geschlechtsspezifischen Eigenschaften des männlichen Blutes und des weiblichen Blutes in der Intensität und im Spektralprofil der registrierten Fluoreszenzstrahlung (51, 52) enthalten sind und wobei zumindest eine der aus den gemessenen spektralen Intensitäten der Fluoreszenzstrahlung (51, 52) ermittelten Intensitätsgrößen oder deren zugeordnete Größen bezüglich des männlichen Blutes gegenüber zumindest einer der ermittelten Intensitätsgrößen oder deren zugeordnete Größen bezüglich des weiblichen Blutes in den Blutgefäßen (22, 24) oder im Herz (25) zumindest einen auswertbaren unterschiedlichen Wert aufweisen,
- eine Bestimmung des Geschlechts des Vogeleies (1) aus dem Unterschied zumindest eines der Werte der Fluoreszenzintensitätsgrößen (51, 52) oder deren zugeordnete Größen und/oder aus einem Vergleich mit einem vorgegebenen Schwellwert (SW) in der Auswerteeinheit (19).

15. Vorrichtung nach Anspruch 14,
**dadurch gekennzeichnet,**
**dass** der Laserstrahl (34), der im Laser (3) produziert wird, mittels Spiegeln oder mittels Faseroptik übertragen wird, wobei der Laserstrahl (34) mit einem Kollimator (4) gebündelt wird.

16. Vorrichtung nach Anspruch 14,
**dadurch gekennzeichnet,**
**dass**
die Vorrichtung (6) zur Einbringung von Laserlicht (34) in das Ei (1) mit zwei
oder mehreren Detektoren (8) zur Aufnahme der Fluoreszenzstrahlung (5) in zwei oder mehreren spektralen Bereichen, die selektiert werden mittels vorgegebenen strahlengangzugeordneten Bandpassfiltern (9), in Verbindung steht.

17. Vorrichtung nach Anspruch 14 und 16,
**dadurch gekennzeichnet,**
**dass** die Vorrichtung (6) zur Einbringung von Laserlicht (34) in das Ei (1) mit
- zwei oder mehreren Laserlicht (34) emittierenden Laserquellen (35, 36), - zwei oder mehreren Kollimatoren (41, 42) für jeweils jede Laserstrahlenquelle (35, 36),
- mindestens einem Strahleinkoppler (26) mit jeweils einem für jede Laserstrahlenquelle (35, 36),
- zwei oder mehreren Detektoren (8) zur Aufnahme der Fluoreszenzstrahlung (5) in zwei oder mehreren Spektralbereichen, die selektiert werden mittels angepassten strahlengangzugehörigen Bandpassfiltern (9),
in Verbindung steht.

18. Vorrichtung nach Anspruch 14,
**dadurch gekennzeichnet,**
**dass** die Vorrichtung (6) zur Einbringung von Laserlicht (34) in das Ei (1), mit einem Spektrometer (20) zur Aufnahme der Fluoreszenzstrahlung (5) und der überlagernden Ramanstreustrahlung in Verbindung steht, wobei dem Spektrometer (20) ein Notchfilter (21) vorgeordnet ist.

19. Vorrichtung nach den Ansprüchen 14 bis 18,
**dadurch gekennzeichnet,**
**dass** das Licht im sichtbaren Wellenlängenbereich, mit dem die Strahlungseinrichtung (13) des Visions-Systems zur Bestrahlung zumindest eines Blutgefäßes (22, 24) oder des Herzens (25) vorgesehen ist, grünes Licht darstellt.

20. Vorrichtung nach den Ansprüchen 14 bis 19,
**dadurch gekennzeichnet,**
**dass** der Detektor (14) für Licht (13a) im sichtbaren Wellenlängenbereich oder im grünen Wellenlängenbereich eine Kamera darstellt.

21. Vorrichtung nach den Ansprüchen 14 bis 20,
**dadurch gekennzeichnet,**
**dass** die Vorrichtung (11) zur Separation der Fluoreszenzstrahlung (5, 51, 52) aus der Rückstreustrahlung ein Strahlteiler ist.

22. Vorrichtung nach den Ansprüchen 14 bis 21,
**dadurch gekennzeichnet,**
**dass** in der Auswerteeinheit (19)
- eine Auswertung der Rückstreustrahlung (5, 50, 51, 52) einschließlich der Fluoreszenzstrahlung (5, 51, 52) aus der registrierten spektralen Intensität der Fluoreszenzstrahlung (5, 51, 52) in einem zur Anregungswellenlänge rotverschobenen Spektralbereich, wobei die geschlechtsspezifischen Eigenschaften des männlichen Blutes und des weiblichen Blutes in der Intensität und im Spektralprofil der registrierten Fluoreszenzstrahlung (5, 51, 52) enthalten sind und wobei zumindest eine der aus den gemessenen spektralen Intensitäten der Fluoreszenzstrahlung (5, 51, 52) ermittelten Intensitätsgrößen oder deren zugeordnete Größen bezüglich des männlichen Blutes gegenüber zumindest einer der ermittelten Intensitätsgrößen oder deren zugeordnete Größen bezüglich des weiblichen Blutes in den Blutgefäßen (22, 24) oder des Herzens (25) zumindest einen auswertbaren unterschiedlichen Wert aufweisen,
- eine Bestimmung des Geschlechts des Vogeleies (1) aus dem Unterschied zumindest eines der Werte der Fluoreszenzintensitätsgrößen (51, 52) oder deren zugeordnete Größen und/oder aus einem Vergleich mit einem vorgegebenen Schwellwert (SW) mittels programmtechnischen Mitteln und/oder optionalen, in der Auswerteeinheit (19) vorhandenen logischen Verknüpfungen
erfolgen.

23. Vorrichtung nach den Ansprüchen 14 bis 22,
**dadurch gekennzeichnet,**
**dass** die Auswerteeinheit (19) zur logischen Verknüpfung von Intensitätsgrößen und zugeordneten Größen oder aus den Intensitätsgrößen abgeleiteten Größen programmtechnische Mittel zur Durchführung von mindestens einer logischen Verknüpfung und/oder als Hardware ausgebildete logische Verknüpfungsglieder (AND) zur Durchführung von mindestens einer logischen Verknüpfung aufweist.

## Claims

1. Method for optically determining the sex of fertilized and incubated bird eggs (1) in ovo, including the following steps:
- monitoring the passage of time of the incubation up to the formation of at least one identifiable blood vessel (22, 24) or of the heart (25) with flowing blood,
- creating a hole (2) in the calcareous shell (28) of the bird egg (1) by means of a hole-producing unit,
- searching for the blood vessels (22, 24) forming in the egg (1) or the heart (25) by means of a vision system (13, 13a) and coaxial or lateral illumination with light in the visible wavelength range,
- positioning at least one blood vessel (22, 24) or the heart (25) in the laser focus of at least one laser beam source (3; 35, 36), either by moving the egg (1) or by moving an objective lens (7) producing the laser focus,
- irradiating the blood vessel (22, 24) or the heart (25) with at least one laser radiation source (3, 35, 36) emitting an excitation wavelength,
- registering the backscattered radiation (5, 50, 51, 52) of the irradiated blood vessel (22, 24) or the heart (25) by means of at least one detector (8), which is connected to at least one evaluation unit (19), which is connected to an amplification and detector unit (12) connected downstream, wherein a movement of the blood vessel (22, 24) or of the heart (25) out of the laser beam (3b) during the registration can take place by way of tracking the blood vessels (22, 24) or the heart (25) or the objective lens (7),
**characterized by** the following steps:
- evaluating the backscattered radiation (5, 50, 51, 52) including the fluorescence radiation (5, 51, 52) in the evaluation unit (19) from the registered spectral intensity of the fluorescence radiation (51, 52) in a spectral range that is red-shifted with respect to the excitation wavelength, wherein the sex-specific properties of the male blood and of the female blood are contained in the intensity and in the spectral profile of the registered fluorescence radiation (51, 52) and wherein at least one of the intensity variables determined from the measured spectral intensities of the fluorescence radiation (51, 52) or their assigned variables with respect to the male blood has an evaluable different value in comparison with at least one of the determined intensity variables or their assigned variables with respect to the female blood in the blood vessels (22, 24) or in the heart (25),
- determining the sex of the bird egg (1) from the difference of at least one of the values of the fluorescence intensity variables (51, 52) or their assigned variables in the evaluation unit (19), and subsequently at least
- displaying the sex of the embryo (23) in the bird egg (1) that has been determined in the evaluation unit (19) .

2. Method according to Claim 1,
**characterized**
**in that** green light is used as the light in the visible wavelength range with which a radiation device (13) of the vision system is provided for irradiating at least one blood vessel (22, 24) or the heart (25).

3. Method according to Claim 1,
**characterized**
**in that** the fluorescence intensities I_{I511}, I_{I512}, I_{I521}, I_{I522} are evaluated with one of the following methods or a combination of the following methods in the evaluation unit (19) in order to obtain the sex:
- intensities are compared with threshold values (SW),
- intensity ratios are compared with threshold values (SW),
- intensity sums or differences are compared with threshold values (SW),
- monitored classification methods, such as for example discriminant analyses, support-vector machines or neural networks are applied to the intensity values.

4. Method according to Claim 1,
**characterized**
**in that** the fluorescence intensities are normalized, for example by way of vector or area normalization, and subsequently mathematically analysed with one of the following methods or a combination of the following methods in the evaluation unit (19) in order to obtain the sex:
- normalized intensities are compared with threshold values,
- ratios between normalized intensities are compared with threshold values,
- sums or differences of normalized intensities are compared with threshold values,
- monitored classification methods, such as for example discriminant analyses, support-vector machines or neural networks are applied to the normalized intensity values.

5. Method according to Claim 1,
**characterized**
**in that** the respectively evaluable different value of the intensity variables relates at least to a measured threshold value (SW) or a specified threshold value (SW) which is stored in the evaluation unit (19) and assigned to the intensity variable.

6. Method according to Claim 1,
**characterized**
**in that** the intensity variables determined from the determined fluorescence intensity/wavenumber curves (51, 52) are defined in the evaluation unit (19) with respect to a specified spectral range as the integral intensity I_{I51} of the fluorescence radiation (51) of the male blood and as the integral intensity I_{I52} of the fluorescence radiation (52) of the female blood.

7. Method according to Claim 1,
**characterized**
**in that** the intensity variables determined from the determined fluorescence intensity/wavenumber curves (51, 52) are defined in the evaluation unit (19) with respect to a specified spectral range as the intensity maximum 51_{Max} of the fluorescence radiation (51) of the male blood and as the intensity maximum 52_{Max} of the fluorescence radiation (52) of the female blood.

8. Method according to Claim 1,
**characterized**
**in that** the intensity variables I_{D1} to I_{D8} determined from the determined fluorescence intensity/wavenumber curves (51, 52) are defined in the evaluation unit (19) with respect to a plurality of specified spectral ranges (D1 to D8) arranged one next to the other as the integral intensity of the fluorescence radiation (51) of the male blood and as the integral intensity of the fluorescence radiation (52) of the female blood within the specified spectral range (D1 to D8).

9. Method according to Claims 2 to 8,
**characterized**
**in that**, when using one laser beam source (3), the intensity variables, determined from the determined fluorescence intensity/wavenumber curve (51, 52), are defined in the evaluation unit (19) with respect to a specified spectral range in each case for the male blood and for the female blood by means of a logical link combined by means of a logical link for evaluation purposes.

10. Method according to Claims 1 to 4,
**characterized**
**in that**, when using at least two laser beam sources (35, 36), the intensity variables, determined from a plurality of determined fluorescence intensity/wavenumber curves (51, 52), are defined in the evaluation unit (19) with respect to a specified spectral range as the integral intensities I₁₅₁₁, I_{I512} of the fluorescence radiations (51) of the male blood and as the integral intensities I_{I521}, I_{I522} of the fluorescence radiations (52) of the female blood.

11. Method according to Claims 2 to 10, **characterized**
**in that**, when using at least two laser beam sources (35, 36), the intensity variables, determined from a plurality of determined fluorescence intensity/wavenumber curves (51, 52), are defined in the evaluation unit (19) with respect to a specified spectral range in each case for the male blood and for the female blood combined by means of a logical link for evaluation purposes.

12. Method according to Claim 1,
**characterized**
**in that** the intensity variables, determined from the determined fluorescence intensity/wavenumber curves (51, 52), are defined in the evaluation unit (19) with respect to a specified spectral range as the integral intensity I_{I51} of the fluorescence radiation (51) of the male blood and as the integral intensity I_{I52} of the fluorescence radiation (52) of the female blood, and the Raman scattered radiations (50₅₁, 50₃₂) respectively superposing the fluorescence radiations (51, 52) are defined in the evaluation unit (19) for the evaluation alone or in combination with the other defined intensity variables and assigned variables, wherein, in the case of a combination of the intensity variables and assigned variables, the Raman scattered radiation and the fluorescence radiation are evaluated together by means of a logical link.

13. Method according to Claim 1,
**characterized**
**in that**, when using pulsed laser radiation from the laser beam source (3), the fluorescence intensity produced is measured in a time-resolved manner and the sex is determined from the time constants τ of the decay curve of the time-resolved fluorescence intensity, wherein in each case a different time constant τ_{männlich}, τ_{weiblich} is determined for male blood and for female blood, with τ_{männlich} ≠ τ_{weiblich}, in order to determine the sex.

14. Apparatus (30, 31, 32, 33) for optically determining the sex of fertilized and incubated bird eggs (1) in ovo on the basis of produced backscattered radiation with the use of the method according to Claims 1 to 13, wherein the apparatus (30) comprises:
- an egg storage unit (16), on which the egg (1) is supported,
- a position evaluation device (17), which is connected to the egg storage unit (16),
- a radiation device (13) with light in the visible wavelength range for irradiating at least one blood vessel (22, 24) or the heart (25) of the embryo (23),
- a detector (14) for visible or green light (13a) for identifying at least one blood vessel (22, 24) or the heart (25) of the embryo (23), wherein the detector (14) is connected to the positioning evaluation device (18),
- an apparatus (6) for introducing laser light (34) into the egg (1), at least comprising
a) at least one laser source (3) emitting laser light (34),
b) at least one detector (8) for recording the fluorescence radiation (5),
c) a control unit (17) for xyz-positioning the apparatus (6) onto the hole (2) introduced into the egg (1),
d) a sex determination evaluation unit (19), which is connected to the amplification and detection unit (12) and the positioning evaluation device (18) that is connected to the control unit (17),
**characterized**
**in that** an apparatus (11) for separating the fluorescence radiation (5) from the backscattered radiation is arranged between a collimator (4) for beam focusing of the laser beam (34) and the apparatus for introducing the laser light (34) into the egg (1), wherein in each case a beam-path-associated detection filter (9) for the transmission of the fluorescence radiation (5) is introduced between the beam-separating apparatus (11) and at least one fluorescence detector (8),
wherein the following take place in the sex determination evaluation unit (19):
- evaluation of the backscattered radiation (5, 50, 51, 52) including the fluorescence radiation (5, 51, 52) from the registered spectral intensity of the fluorescence radiation (51, 52) in a spectral range that is red-shifted with respect to the excitation wavelength, wherein the sex-specific properties of the male blood and of the female blood are contained in the intensity and in the spectral profile of the registered fluorescence radiation (51, 52) and wherein at least one of the intensity variables determined from the measured spectral intensities of the fluorescence radiation (51, 52) or their assigned variables with respect to the male blood has at least one evaluable different value in comparison with at least one of the determined intensity variables or their assigned variables with respect to the female blood in the blood vessels (22, 24) or in the heart (25),
- a determination of the sex of the bird egg (1) from the difference of at least one of the values of the fluorescence intensity variables (51, 52) or their assigned variables and/or from a comparison with a specified threshold value (SW) in the evaluation unit (19) .

15. Apparatus according to Claim 14,
**characterized**
**in that** the laser beam (34) produced in the laser (3) is transmitted by means of mirrors or by means of a fibre-optic unit, wherein the laser beam (34) is focused using a collimator (4).

16. Apparatus according to Claim 14,
**characterized**
**in that** the apparatus (6) for introducing laser light (34) into the egg (1) is connected to two or more detectors (8) for recording the fluorescence radiation (5) in two or more spectral ranges, which are selected by means of specified, beam-path-associated bandpass filters (9).

17. Apparatus according to Claim 14 and 16, **characterized**
**in that** the apparatus (6) for introducing laser light (34) into the egg (1) is connected to
- two or more laser sources (35, 36) emitting laser light (34),
- two or more collimators (41, 42) for each respective laser beam source (35, 36),
- at least one beam coupling-in device (26) with in each case one for each laser radiation source (35, 36),
- two or more detectors (8) for recording the fluorescence radiation (5) in two or more spectral ranges, which are selected by means of adapted beam-path-associated bandpass filters (9).

18. Apparatus according to Claim 14,
**characterized**
**in that** the apparatus (6) for introducing laser light (34) into the egg (1) is connected to a spectrometer (20) for recording the fluorescence radiation (5) and the superposing Raman scattered radiation, wherein a notch filter (21) is arranged upstream of the spectrometer (20) .

19. Apparatus according to Claims 14 to 18, **characterized**
**in that** the light in the visible wavelength range with which the irradiation device (13) of the vision system is provided for irradiating at least one blood vessel (22, 24) or the heart (25) is green light.

20. Apparatus according to Claims 14 to 19, **characterized**
**in that** the detector (14) for light (13a) in the visible wavelength range or in the green wavelength range is a camera.

21. Apparatus according to Claims 14 to 20, **characterized**
**in that** the apparatus (11) for separating the fluorescence radiation (5, 51, 52) from the backscattered radiation is a beam splitter.

22. Apparatus according to Claims 14 to 21, **characterized**
**in that** the following take place in the evaluation unit (19) :
- an evaluation of the backscattered radiation (5, 50, 51, 52) including the fluorescence radiation (5, 51, 52) from the registered spectral intensity of the fluorescence radiation (5, 51, 52) in a spectral range which is red-shifted with respect to the excitation wavelength, wherein the sex-specific properties of the male blood and of the female blood are contained in the intensity and in the spectral profile of the registered fluorescence radiation (5, 51, 52) and wherein at least one of the intensity variables determined from the measured spectral intensities of the fluorescence radiation (5, 51, 52) or their assigned variables with respect to the male blood has at least one evaluable different value in comparison with at least one of the determined intensity variables or their assigned variables with respect to the female blood in the blood vessels (22, 24) or in the heart (25),
- a determination of the sex of the bird egg (1) from the difference of at least one of the values of the fluorescence intensity variables (51, 52) or their assigned variables and/or from a comparison with a specified threshold value (SW) by means of program-technological means and/or optional logical links present in the evaluation unit (19).

23. Apparatus according to Claims 14 to 22, **characterized**
**in that** the evaluation unit (19) has, for the logical link of intensity variables and assigned variables or variables derived from the intensity variables, program-technological means for carrying out at least one logical link and/or logical link operators (AND), in the form of hardware, for carrying out at least one logical link.

## Revendications

1. Procédé de sexage optique in ovo d'œufs d'oiseau fécondés et incubés (1), comprenant les étapes suivantes consistant à :
- surveiller l'évolution de l'incubation jusqu'à la formation d'au moins un vaisseau sanguin identifiable (22, 24) ou du cœur (25) avec du sang qui circule,
- pratiquer un trou (2) dans la coquille calcaire (28) de l'œuf d'oiseau (1) au moyen d'une unité de génération de trou,
- rechercher les vaisseaux sanguins (22, 24) qui se forment dans l'œuf (1) ou le cœur (25) au moyen d'un système de vision (13, 13a) et d'un éclairage coaxial ou latéral avec de la lumière dans le domaine des longueurs d'onde visibles,
- positionner au moins un vaisseau sanguin (22, 24) ou le cœur (25) dans le foyer laser d'au moins une source de faisceau laser (3 ; 35, 36) soit en déplaçant l'œuf (1), soit en déplaçant un objectif (7) générant le foyer laser,
- exposer le vaisseau sanguin (22, 24) ou le cœur (25) à un rayonnement par au moins une source de faisceau laser (3, 35, 36) émettant une longueur d'onde d'excitation,
- enregistrer le rayonnement rétrodiffusé (5, 50, 51, 52) du vaisseau sanguin (22, 24) ou du cœur (25) exposé au rayonnement au moyen d'au moins un détecteur (8) qui est en communication avec au moins une unité d'évaluation (19) reliée à une unité d'amplification et de détection (12) placée en aval, dans lequel, pendant l'enregistrement, un déplacement du vaisseau sanguin (22, 24) ou du cœur (25) hors du faisceau laser (3b) peut être effectué par une poursuite des vaisseaux sanguins (22, 24) ou du cœur (25) ou de l'objectif (7), **caractérisé par** les étapes suivantes consistant à :
- évaluer le rayonnement rétrodiffusé (5, 50, 51, 52), y compris le rayonnement fluorescent (5, 51, 52) dans l'unité d'évaluation (19) provenant de l'intensité spectrale enregistrée du rayonnement fluorescent (51, 52), dans un domaine spectral décalé vers le rouge par rapport à la longueur d'onde d'excitation, les propriétés spécifiques au sexe du sang masculin et du sang féminin étant contenues dans l'intensité et dans le profil spectral du rayonnement fluorescent enregistré (51, 52), et dans lequel au moins l'une des grandeurs d'intensité établies à partir des intensités spectrales mesurées du rayonnement fluorescent (51, 52) ou leurs grandeurs associées concernant le sang masculin présentent une valeur différente exploitable par rapport à au moins l'une des grandeurs d'intensité établies ou à leurs grandeurs associées concernant le sang féminin dans les vaisseaux sanguin (22, 24) ou dans le cœur (25),
- déterminer le sexe de l'œuf d'oiseau (1) à partir de la différence d'au moins l'une des valeurs des grandeurs d'intensité de fluorescence (51, 52) ou de leurs grandeurs associées dans l'unité d'évaluation (19), et ensuite au moins
- afficher le sexe de l'embryon (23) dans l'œuf d'oiseau (1) déterminé dans l'unité d'évaluation (19).

2. Procédé selon la revendication 1, **caractérisé en ce que** de la lumière verte est utilisée comme lumière dans le domaine des longueurs d'onde visibles, avec laquelle un dispositif de rayonnement (13) du système de vision est prévu pour exposer au moins un vaisseau sanguin (22, 24) ou le cœur (25) au rayonnement.

3. Procédé selon la revendication 1, **caractérisé en ce que** les intensités de fluorescence I_{I511}, I_{I512}, I_{I521}, I_{I522} sont évaluées par l'un des procédés suivants ou une combinaison des procédés suivants dans l'unité d'évaluation (19) afin d'obtenir le sexe :
(SW), - comparer les intensités avec des valeurs seuil
- comparer les rapports d'intensités avec des valeurs seuil (SW),
- comparer des sommes ou des différences d'intensités avec des valeurs seuil (SW),
- appliquer des méthodes de classification surveillées, comme par exemple des analyses discriminantes, des machines à vecteurs de support ou des réseaux neuronaux, aux valeurs d'intensité normalisées.

4. Procédé selon la revendication 1, **caractérisé en ce que** les intensités de fluorescence sont normalisées, par exemple par normalisation vectorielle ou de surface, en sont ensuite évaluées mathématiquement par l'une des méthodes suivantes ou une combinaison des procédés suivants dans l'unité d'évaluation (19) afin d'obtenir le sexe :
- comparer des intensités normalisées avec des valeurs seuil,
- comparer des rapports entre des intensités normalisées avec des valeurs seuil,
- comparer des sommes ou des différences des intensités normalisées avec des valeurs seuil,
- appliquer des méthodes de classification surveillées, comme par exemple des analyses discriminantes, des machines à vecteurs de support ou des réseaux neuronaux, aux valeurs d'intensités normalisées.

5. Procédé selon la revendication 1, **caractérisé en ce que** la valeur différente exploitable respectivement des grandeurs d'intensité fait référence au moins à une valeur seuil mesurée (SW) ou à une valeur seuil prédéfinie, associée à la grandeur d'intensité (SW) et stockée dans l'unité d'évaluation (19).

6. Procédé selon la revendication 1, **caractérisé en ce que** dans l'unité d'évaluation (19), les grandeurs d'intensité établies à partir des courbes intensité de fluorescence/ nombre d'ondes (51, 52) établies concernant un domaine spectral prédéfini sont définies comme l'intensité intégrale I_{I51} du rayonnement fluorescent (51) du sang masculin et comme l'intensité intégrale I_{I52} du rayonnement fluorescent (52) du sang féminin.

7. Procédé selon la revendication 1, **caractérisé en ce que** dans l'unité d'évaluation (19), les grandeurs d'intensité établies à partir des courbes intensité de fluorescence/ nombre d'ondes établies (51, 52) concernant un domaine spectral prédéfini sont définies comme le maximum d'intensité 51_{Max} du rayonnement fluorescent (51) du sang masculin et comme le maximum d'intensité 52_{Max} du rayonnement fluorescent (52) du sang féminin.

8. Procédé selon la revendication 1, **caractérisé en ce que** dans l'unité d'évaluation (19), les grandeurs d'intensité I_{D1} à I_{D8} établies à partir des courbes intensité de fluorescence/ nombre d'ondes établies (51, 52) concernant plusieurs domaines spectraux (D1 à D8) prédéfinis, disposés côte à côte, sont définies comme l'intensité intégrale du rayonnement fluorescent (51) du sang masculin et comme l'intensité intégrale du rayonnement fluorescent (52) du sang féminin à l'intérieur des domaines spectraux prédéfinis (D1 à D8).

9. Procédé selon les revendications 2 à 8, **caractérisé en ce que** dans l'unité d'évaluation (19), lorsqu'une source de faisceau laser (3) est mise en œuvre, les grandeurs d'intensité établies à partir de la courbe intensité de fluorescence/ nombre d'ondes établie (51, 52) concernant un domaine spectral prédéfini sont définies pour l'évaluation en combinaison respectivement pour le sang masculin et pour le sang féminin au moyen d'une combinaison logique au moyen d'une combinaison logique.

10. Procédé selon les revendications 1 à 4, **caractérisé en ce que** dans l'unité d'évaluation (19), lorsqu'au moins deux sources de faisceau laser (35, 36) sont mises en œuvre, les grandeurs d'intensité établies à partir de plusieurs courbes intensité de fluorescence/ nombre d'ondes établies (51, 52) concernant un domaine spectral prédéfini sont définies comme les intensités intégrales I₁₅₁₁, I_{I512} des rayonnements fluorescents (51) du sang masculin et comme les intensités intégrales I_{I521}, I_{I522} des rayonnements fluorescents (52) du sang féminin.

11. Procédé selon les revendications 2 à 10, **caractérisé en ce que** dans l'unité d'évaluation (19), lorsqu'au moins deux sources de faisceau laser (35, 36) sont mises en œuvre, les grandeurs d'intensité établies à partir de plusieurs courbes intensité de fluorescence/ nombre d'ondes établies (51, 52) concernant un domaine spectral prédéfini sont définies pour l'évaluation en combinaison respectivement pour le sang masculin et pour le sang féminin au moyen d'une combinaison logique.

12. Procédé selon la revendication 1, **caractérisé en ce que** dans l'unité d'évaluation (19), les grandeurs d'intensité établies à partir des courbes intensité de fluorescence/ nombre d'ondes établies (51, 52) concernant un domaine spectral prédéfini sont définies comme l'intensité intégrale I_{I51} du rayonnement fluorescent (51) du sang masculin et comme l'intensité intégrale I_{I52} du rayonnement fluorescent (52) du sang féminin, et les rayonnements à diffusion Raman (50₅₁, 50₅₂) respectivement superposés aux rayonnements fluorescents (51, 52) sont définis pour l'évaluation seuls ou en combinaison avec les autres grandeurs d'intensité définies et des grandeurs associées, dans lequel, lors de la combinaison des grandeurs d'intensité et des grandeurs associées, le rayonnement à diffusion Raman et le rayonnement fluorescent sont évalués ensemble au moyen d'une combinaison logique.

13. Procédé selon la revendication 1, **caractérisé en ce que**, lorsqu'un rayonnement laser pulsé est mis en œuvre, l'intensité de fluorescence générée est mesurée de manière résolue dans le temps à partir de la source de faisceau laser (3), et un sexage est effectué à partir de la constante de temps T de la courbe d'évanouissement de l'intensité de fluorescence résolue dans le temps, dans lequel, pour le sang masculin et pour le sang féminin, respectivement une constante de temps différente, τ_{männlich}, τ_{weiblich}, où τ_{männlich} ≠ τ_{weiblich}, est établie pour le sexage.

14. Dispositif (30, 31, 32, 33) de sexage optique in ovo d'œufs d'oiseau fécondés et incubés (1) sur la base d'un rayonnement rétrodiffusé créé en utilisant le procédé selon les revendications 1 à 13, le dispositif (30) comprenant
- une unité de logement d'œuf (16) sur laquelle est posé l'œuf (1),
- un moyen d'évaluation de position (17) qui est en communication avec l'unité de logement d'œuf (16),
- un moyen de rayonnement (13) avec de la lumière dans le domaine des longueurs d'onde visibles pour exposer au moins un vaisseau sanguin (22, 24) ou le cœur (25) de l'embryon (23) à un rayonnement,
- un détecteur (14) pour la lumière visible ou verte (13a) servant à identifier au moins un vaisseau sanguin (22, 24) ou le cœur (25) de l'embryon (23), le détecteur (14) étant en communication avec le moyen d'évaluation de positionnement (18),
- un dispositif (6) servant à introduire de la lumière laser (34) dans l'œuf (1), qui comprend au moins
a) au moins une source laser (3) émettant de la lumière laser (34),
b) au moins un détecteur (8) servant à recevoir le rayonnement fluorescent (5),
c) une unité de commande (17) servant au positionnement xyz du dispositif (6) sur le trou (2) pratiqué dans l'œuf (1),
d) une unité d'évaluation de sexage (19) qui est en communication avec l'unité d'amplification et de détection (12) et le moyen d'évaluation de positionnement (18) qui est relié à l'unité de commande (17),
**caractérisé en ce qu'**un dispositif (11) servant à séparer le rayonnement fluorescent (5) du rayonnement rétrodiffusé est disposé entre un collimateur (4) pour la focalisation du faisceau laser (34) et le dispositif servant à introduire la lumière laser (34) dans l'œuf (1), dans lequel, entre le dispositif de séparation de faisceau (11) et au moins un détecteur de fluorescence (8), respectivement un filtre de détection (9) associé à une trajectoire de faisceau est introduit pour la transmission du rayonnement fluorescent (5),
dans lequel, dans l'unité d'évaluation de sexage (19), les étapes suivantes sont effectuées
- une évaluation du rayonnement rétrodiffusé (5, 50, 51, 52), y compris le rayonnement fluorescent (5, 51, 52) provenant de l'intensité spectrale enregistrée du rayonnement fluorescent (51, 52), dans un domaine spectral décalé vers le rouge par rapport à la longueur d'onde d'excitation, les propriétés spécifiques au sexe du sang masculin et du sang féminin étant contenues dans l'intensité et dans le profil spectral du rayonnement fluorescent enregistré (51, 52), et dans lequel au moins l'une des grandeurs d'intensité établies à partir des intensités spectrales mesurées du rayonnement fluorescent (51, 52) ou leurs grandeurs associées concernant le sang masculin présentent au moins une valeur différente exploitable par rapport à au moins l'une des grandeurs d'intensité établies ou à leurs grandeurs associées concernant le sang féminin dans les vaisseaux sanguins (22, 24) ou dans le cœur (25),
- une détermination du sexe de l'œuf d'oiseau (1) à partir de la différence d'au moins l'une des valeurs des grandeurs d'intensité de fluorescence (51, 52) ou de leurs grandeurs associées et/ou d'une comparaison avec une valeur seuil prédéfinie (SW) dans l'unité d'évaluation (19).

15. Dispositif selon la revendication 14, **caractérisé en ce que** le faisceau laser (34) qui est produit dans le laser (3) est transmis au moyen de miroirs ou au moyen de fibres optiques, le faisceau laser (34) étant focalisé par un collimateur (4).

16. Dispositif selon la revendication 14, **caractérisé en ce que** le dispositif (6) servant à introduire de la lumière laser (34) dans l'œuf (1) est en communication avec deux ou plusieurs détecteurs (8) servant à capter le rayonnement fluorescent (5) dans deux ou plusieurs domaines spectraux qui sont sélectionnés au moyen de filtres passe-bande (9) prédéfinis, associés à des trajectoires de faisceau.

17. Dispositif selon les revendications 14 et 16, **caractérisé en ce que** le dispositif (6) servant à introduire de la lumière laser (34) dans l'œuf (1) est en communication avec
- deux ou plusieurs sources laser (35, 36) émettant de la lumière laser (34),
- deux ou plusieurs collimateurs (41, 42) pour respectivement chaque source de faisceau laser (35, 36),
- au moins un coupleur de faisceau (26) avec respectivement un pour chaque source de faisceau laser (35, 36),
- deux ou plusieurs détecteurs (8) servant à capter le rayonnement fluorescent (5) dans deux ou plusieurs domaines spectraux qui sont sélectionnés au moyen de filtres passe-bande (9) adaptés, associés à des trajectoires de faisceau.

18. Dispositif selon la revendication 14, **caractérisé en ce que** le dispositif (6) servant à introduire de la lumière laser (34) dans l'œuf (1) est en communication avec un spectromètre (20) pour capter le rayonnement fluorescent (5) et le rayonnement à diffusion Raman superposé, un filtre éliminateur (21) étant placé en amont du spectromètre (20).

19. Dispositif selon les revendications 14 à 18, **caractérisé en ce que** la lumière dans le domaine des longueurs d'onde visibles, avec laquelle le moyen de rayonnement (13) du système de vision est prévu pour exposer au moins un vaisseau sanguin (22, 24) ou le cœur (25) à un rayonnement, est de la lumière verte.

20. Dispositif selon les revendications 14 à 19, **caractérisé en ce que** le détecteur (14) pour la lumière (13a) dans le domaine des longueurs d'onde visibles ou dans le domaine des longueurs d'onde vertes est une caméra.

21. Dispositif selon les revendications 14 à 20, **caractérisé en ce que** le dispositif (11) servant à séparer le rayonnement fluorescent (5, 51, 52) du rayonnement rétrodiffusé est un séparateur de faisceau.

22. Dispositif selon les revendications 14 à 21, **caractérisé en ce que** dans l'unité d'évaluation (19), les étapes suivantes sont effectuées
- une évaluation du rayonnement rétrodiffusé (5, 50, 51, 52), y compris le rayonnement fluorescent (5, 51, 52) provenant de l'intensité spectrale enregistrée du rayonnement fluorescent (5, 51, 52), dans un domaine spectral décalé vers le rouge par rapport à la longueur d'onde d'excitation, les propriétés spécifiques au sexe du sang masculin et du sang féminin étant contenues dans l'intensité et dans le profil spectral du rayonnement fluorescent enregistré (5, 51, 52), et dans lequel au moins l'une des grandeurs d'intensité établies à partir des intensités spectrales mesurées du rayonnement fluorescent (5, 51, 52) ou leurs grandeurs associées concernant le sang masculin présentent au moins une valeur différente exploitable par rapport à au moins l'une des grandeurs d'intensité établies ou à leurs grandeurs associées concernant le sang féminin dans les vaisseaux sanguins (22, 24) ou dans le cœur (25),
- une détermination du sexe de l'œuf d'oiseau (1) à partir de la différence d'au moins l'une des valeurs des grandeurs d'intensité de fluorescence (51, 52) ou de leurs grandeurs associées et/ou d'une comparaison avec une valeur seuil prédéfinie (SW) à l'aide de moyens logiciels et/ou de combinaisons logiques en option, présentes dans l'unité d'évaluation (19).

23. Dispositif selon les revendications 14 à 22, **caractérisé en ce que** l'unité d'évaluation (19) présente pour la combinaison logique de grandeurs d'intensité et de grandeurs associées ou de grandeurs dérivées des grandeurs d'intensité des moyens logiciels servant à exécuter au moins une combinaison logique et/ou des éléments de combinaison logique (ET) réalisés sous forme matérielle servant à exécuter au moins une combinaison logique.
